# EUROPEAN PATENT SPECIFICATION

(11) **EP 3 540 013 B1**
(45) Date of publication and mention of the grant of the patent: **25.05.2022**
(21) Application number: 19172014.3
(22) Date of filing: 14.04.2015
(51) Int. Cl.: C08L 81/00, C08G 75/045

(54) **RESIN COMPOSITION SUITABLE FOR PRINTING AND PRINTING METHOD UTILIZING THE SAME**
ZUM DRUCKEN GEEIGNETE HARZZUSAMMENSETZUNG UND DRUCKVERFAHREN DAMIT
COMPOSITION DE RÉSINE APPROPRIÉE POUR L'IMPRESSION ET PROCÉDÉ D'IMPRESSION L'UTILISANT

(30) Priority: 14.04.2014 GB 201406683
(43) Date of publication of application: 18.09.2019
(62) Divisional of application: 15719633.8
(73) Proprietor: Montanuniversität Leoben, 8700 Leoben (AT)
(72) Inventor: Oesterreicher, Andreas, 6313 Edlibach (CH); Griesser, Thomas, 8792 St. Peter-Freienstein (AT); Edler, Matthias, 8047 Graz (AT); Mostegel, Florian, 8010 Graz (AT); Gassner, Martina, 8793 Trofaiach (AT); Roth, Meinhart, 86633 Neuburg (DE); Billiani, Janine, 8010 Graz (AT)
(74) Representative: Dilg, Haeusler, Schindelmann Patentanwaltsgesellschaft mbH

(56) References cited:
- WO-A1-2010/141274
- WO-A1-2015/158718
- WO-A2-2009/132070
- US-A- 3 725 228
- FAIRBANKS BENJAMIN ET AL: "Thiol-Yne Photopolymerizations: Novel Mechanism, Kinetics, and Step-Growth Formation of Highly Cross-Linked Networks", ACS MACRO LETTERS, , vol. 42, no. 1 13 January 2009 (2009-01-13), pages 211-217, XP009514622, ISSN: 2161-1653, DOI: 10.1021/MA801903W Retrieved from the Internet: URL:http://pubs.acs.org/doi/abs/10.1021/ma 801903w [retrieved on 2008-12-10]
- HOOGENBOOM RICHARD: "Thiol-Yne Chemistry: A Powerful Tool for Creating Highly Functional Materials", ANGEWANDTE CHEMIE, INTERNATIONAL EDITION, WILEY-VCH, DE , vol. 49, no. 20 3 May 2010 (2010-05-03), pages 3415-3417, XP009514641, ISSN: 1433-7851, DOI: 10.1002/ANIE.201000401 Retrieved from the Internet: URL:https://api.wiley.com/onlinelibrary/td m/v1/articles/10.1002%2Fanie.201000401 [retrieved on 2010-04-14]
- LOWE ANDREW B ET AL: "Thiol-yne click chemistry: A powerful and versatile methodology for materials synthesis", JOURNAL OF MATERIALS CHEMISTRY, ROYAL SOCIETY OF CHEMISTRY, GB , vol. 20, no. 23 1 January 2010 (2010-01-01), pages 4745-4750, XP009514614, ISSN: 0959-9428, DOI: 10.1039/B917102A Retrieved from the Internet: URL:http://xlink.rsc.org/?DOI=b917102a [retrieved on 2010-02-19]
- HOOGENBOOM RICHARD: "Thiol-Yne Chemistry: A Powerful Tool for Creating Highly Functional Materials", ANGEWANDTE CHEMIE, INTERNATIONAL EDITION, WILEY-VCH, DE , vol. 49, no. 20 3 May 2010 (2010-05-03), pages 3415-3417, XP009514641, ISSN: 1433-7851, DOI: 10.1002/ANIE.201000401 Retrieved from the Internet: URL:https://api.wiley.com/onlinelibrary/td m/v1/articles/10.1002%2Fanie.201000401 [retrieved on 2010-04-14]

## Description

### FIELD OF THE INVENTION

The present invention relates to a resin composition, in particular suitable for printing, a kit comprising the components of the resin composition, a printing method utilizing the resin composition, a polymer obtained by the printing method, an article comprising or formed from the polymer, and uses thereof.

### BACKGROUND

Printing methods may serve for a large variety of purposes. In addition to traditional two-dimensional printing methods that are still widely used, three-dimensional printing methods have gained increasing interest over the past years. Initially developed for preparing prototypes, for instance for design purposes, three-dimensional (3D) printing methods are now more and more utilized for producing highly sophisticated and complex geometric structures that are brought to market as such, for instance in the automobile industry, for producing ornaments and in end-user appliances (Wendel et al., Macromol. Mater. Eng. 293 (2008) 799-809).

Among the known 3D printing methods, so-called solid free-form techniques or rapid prototyping, such as fused deposition modeling (FDM), selective laser sintering (SLS) and 3D printing technology (3DP), in particular stereolithography (SLA) is a very promising technique in view of its high resolution that can hardly be attained with other techniques (Gross et al., Anal. Chem. 86 (2014) 3240-3253; Liska et al., J. Coat. Technol. Res. 4 (2007) 505-510). This technique based on photopolymerization is in particular suitable for preparing biomedical products, such as artificial, biologically degradable bone structures requiring pore sizes between 100 µm and 500 µm (Lipowiecki et al., J. Biomed. Mater. Res. Part A (2014) accepted; Bose et al., Materials Today 16 (2013) 496-504). Previous bone replacement materials prepared by FDM, SLS or 3DP are mainly based on ceramic materials, such as calcium phosphate or hydroxyapatite, which however suffer from a low mechanical load capacity with increasing porosity.

Typical biologically degradable polymers used as suture material, in particular for surgical suture, are based on polyester polymers, for instance polycaprolactones (e.g. poly(ε-caprolactone)) and poly(a-hydroxy acids) (e.g. polyglycolic acid, polylactic acid and copolymers thereof). However, due to their relatively fast hydrolytic degradation resulting in loss of their mechanical properties, these materials cannot be used as a substitute for hard tissue. In addition, the high concentration of acid released in the course of the degradation may lead to tissue necrosis (Schuster et al, Proceedings LPM 2007 - The 8th Symposium on Laser Precision Microfabrication).

Among the photo-reactive biologically degradable polymers, poly(propylene fumarate) and poly(trimethylen carbonate-co-caprolactone)-coumarin have proven to be appropriate for preparing porous structures by means of SLA (Melchels et al., Biomaterials 31 (2010) 6121-6130; Cooke et al., J. Biomed. Mater. Res. Part B: Appl. Biomater. 64B (2003) 65-69; Lee et al., Biomacromolecules 8 (2007) 1077-1084; Matsuda et al., Macromolecules 33 (2000) 795-800). Microneedles prepared by SLA from acrylated poly(trimethylen carbonate-co-caprolactone) have been subcutaneously implanted in rats for one month and have shown a regular degradation by surface erosion similar to that of an alkaline hydrolysis (Matsuda et al., J. Biomed. Mater. Res. 62 (2002) 395-403). Liska et al. evaluated a variety of photo-reactive polymers as resins used for stereolithography and two-photon absorption (TPA) photopolymerization, respectively, for preparing cellular structures. The monomers mainly based on acrylates, methacrylates as well as acrylamides have proven to be non-cytotoxic and could be structured by stereolithographic methods by means of digital light processing (PLP) or UV laser microstereolithography with a resolution of 5 to 10 µm (Liska et al., J. Coat. Technol. Res. 4 (2007) 505-510; Schuster et al, Proceedings LPM 2007 - The 8th Symposium on Laser Precision Microfabrication).

Despite these efforts, the choice of commercially available printing resins is however still limited so that there is a need for the development of new materials and compositions.

Thiol-ene reactions represent an established photopolymerization process used for a variety of appliances, in particular in the optical, biomedical, bioorganic and dental field (Hoyle et al., Angew. Chem. Int. Ed. 49 (2010) 5301-5338). Advantages of thiol-ene reactions reside in a high reactivity, insensitivity against oxygen, high chemical stability, low shrinkage of the obtained polymers as well as high polymerization yields (Hoyle et al., J. Polym. Sci. Part A: Polym. Chem. 42 (2004) 5301-5338). WO 2013/052328 A1 discloses resorbable biocompatible polymers formed by thiol-ene polymerization of vinylesters and vinylcarbonates, which polymers can be used as a biodegradable, resorbable implant.

Thiol-yne photopolymerizations run with a similar mechanism to thiol-ene reactions, and thus they possess the same advantages. However, polymers obtained by thiol-yne reactions have a six-time higher crosslinking degree and consequently a significantly higher glass transition temperature than corresponding thiol-ene based polymers (Fairbanks et al., Macromolecules 42 (2009) 211-217). Due to the ability of an alkyne moiety to react with two thiol monomers, monofunctional monomers, such as compounds having only one alkyne moiety, may be used in thiol-yne reactions without terminating the polymerization reaction. Thiol-yne based polymers possess high biocompatibility and are thus suitable for the biomedical field. WO 2012/103445 A2 discloses the preparation of biodegradable hydrogels on the basis of a thiol-yne reaction, which hydrogels are described to be suitable for biomedicine, for instance as a substrate for the growth of various cell types. US 2011/0144227 A1 discloses biomedical devices containing thiol-ene or thiol-yne shape memory polymers. A study about thiol-yne chemistry as a powerful tool for creating highly functional materials is disclosed in Hoogenboom, Angew. Chem. Int. Ed. 49 (2010) 3415-3417 and an overview over thiol-yne click chemistry is given by Lowe et al. in J: Mater: Chem. 20 (2010) 4745-4750. WO 2010/141274 A1 discloses thiol-yne shape memory polymer compositions comprising an alkynyl compound having at least one reactive alkynyl group, a polythiol and less than 2 wt.% of a free radical initiator. US 3,725,228 discloses high energy curable compositions comprising a terminally unsaturated polyene component and a polythiol component.

### OBJECTS OF THE INVENTION

In light of the foregoing, the present invention aims at overcoming the above described problems and drawbacks of hitherto available printing resins, in particular the restrictions thereof when used for medical or biomedical appliances. Thus, an object of the present invention is to provide a novel resin composition which may be in particular suitable for printing and which benefits from the advantages of thiol-yne polymerization reactions. Another object is to provide a polymer and an article comprising or formed from the polymer, which is highly biocompatible, has a very low residual monomer content and exhibits low shrinkage.

### SUMMARY OF THE INVENTION

The present inventors have made diligent studies for solving these objects and have found that when the alkyne functional group-containing component in a resin composition further comprises at least one functional group selected from a carbonate, a carbamate and/or an ether, in particular one functional carbonate group, not only a printing resin having highly biocompatible monomers and being excellently suitable for a 3D printing method, such as stereolithography, with high curing velocity of the resin, very high monomer conversion (thereby substantially avoiding any migration of monomers from the final product) and insensitivity to oxygen during polymerization can be provided, but also the biodegradability of the resulting polymer as well as of an article comprising or formed from the polymer can be appropriately adjusted to the desired needs of a specific medical or biomedical appliance and the shrinkage of the resulting polymer is low due to the formation of a homogenous network as well as the mechanical properties, such as the elastic modulus, are excellent due to its high crosslinking degree, which properties are particularly beneficial in medical or biomedical appliances, such as implantation, bone substitution or replacement, tissue substitution or replacement and/or dental applications. In addition, the composition and a printing method utilizing the composition may be solvent-free which further improves the biocompatibility of the resulting products due to the absence of any residual solvent. Moreover, additives may be included in the composition, which additives may provide for additional specific tailor-made advantageous properties of the resulting products, as desired.

Accordingly, the present invention relates to a resin composition comprising:
at least one compound C1 having
   (i) two terminal alkyne functional groups selected from the group consisting of propargyl, butynyl and pentynyl, and
   (ii) one functional carbonate group; and
at least one compound C2 having at least two thiol functional groups.

The resin composition according to the present invention is in particular suitable for printing.

Accordingly, the present invention further relates to the use of the resin composition according to the present invention as or in an ink.

The components C1 and C2 of the resin composition according to the present invention may be in particular provided in a spatially separated manner, for instance in a kit, in particular a kit-of-parts.

Thus, the present invention also relates to a kit comprising:
at least one compound C1 having
   (i) two terminal alkyne functional groups selected from the group consisting of propargyl, butynyl and pentynyl, and
   (ii) one functional carbonate group; and
at least one compound C2 having at least two thiol functional groups.

Furthermore, the present invention relates to the use of the kit according to the present invention for preparing a resin composition for use as or in an ink.

The present invention further relates to a printing method comprising the steps of
providing the resin composition according to the present invention, wherein the resin composition further comprises at least one initiator; and
directing an energy source to at least a part of the resin composition so as to cause polymerization of the at least a part of the resin composition and so as to obtain a polymer.

In addition, the present invention relates to a polymer obtainable by the printing method according to the present invention.

Moreover, the present invention relates to an article comprising or formed from the polymer according to the present invention.

The polymer and the article obtained according to the present invention can be used for various appliances.

Accordingly, the present invention further relates to the use of the polymer or of the article according to the present invention in a medical or biomedical application.

Other objects and many of the attendant advantages of embodiments of the present invention will be readily appreciated and become better understood by reference to the following detailed description of embodiments and the accompanying drawings.

### BRIEF DESCRIPTION OF THE DRAWINGS

Figure 1 is a graphical illustration of the test results of Example 1 indicating the reaction speeds of the tested alkyne monomers and comparative (meth)acryl monomers and further showing their chemical structural formulas.
Figure 2 is a graphical three-dimensional illustration of the temporal decrease of the alkyne peak (at 3280 cm⁻¹) of triethyleneglycol dibut-1-ynyl ether according to Example 2.
Figure 3 is a graphical three-dimensional illustration of the temporal decrease of the thiol peak (at 2565 cm⁻¹) of TMPMP in a thiol-yne reaction with triethyleneglycol dibut-1-ynyl ether according to Example 2.
Figure 4 is a graphical three-dimensional illustration of the temporal decrease of the alkene band (at 1640 cm⁻¹) of 1,4-butanediol diacrylate according to Example 2.
Figure 5 is a graphical illustration of the temporal decrease of the content of alkyne and alkene, respectively, during photopolymerisation of 1,4-butanediol diacrylate, 1,4-triethyleneglycol dibut-1-ynyl ether and 1,4-butanediol dipropargyl carbonate obtained by means of real-time FTIR measurements according to Example 2.
Figure 6 is a graphical illustration of the temporal decrease of the content of alkyne and alkene, respectively, during photopolymerisation of 1,4-butanediol dimethacrylate, 1,4-butanediol dibut-1-ynyl ether and 1,4-butanediol dipropargyl ether obtained by means of real-time FTIR measurements according to Example 2.
Figure 7 shows illustrations of the concentration-dependent cytotoxicity after incubation for 48 h of 1,4-butanediol diacrylate (A), 1,4-butanediol dibut-1-ynyl ether (B), 1,4-butanediol dimethacrylate (C), 1,4-butanediol dipropargyl ether (D) and 1,4-butanediol dipropargyl carbonate (E).

### DETAILLED DESCRIPTION OF THE INVENTION

Hereinafter, details of the present invention and other features and advantages thereof will be described. However, the present invention is not limited to the following specific descriptions, but they are rather for illustrative purposes only.

In a first aspect, the present invention relates to a resin composition comprising:
at least one compound C1 having
   (i) two terminal alkyne functional groups selected from the group consisting of propargyl, butynyl and pentynyl, and
   (ii) one functional carbonate group; and
at least one compound C2 having at least two thiol functional groups.

The term "composition", as used herein, may in particular mean that the components (ingredients) of the composition are in close proximity with each other and/or that the components are (intensely) mixed with each other, for instance by using a mixer, a stirrer and/or by shaking, to thereby form the composition. In particular, the components of the compositions may be uniformly distributed or dispersed throughout within the composition. The composition may be in particular solid, semi-solid (pasty) or liquid, in particular a liquid solution or a semi-solid or liquid suspension.

The resin composition may in particular be a photo-reactive and/or a thermo-reactive resin composition. The term "photo-reactive", as used herein, may in particular mean that the resin composition, in particular some or all of its components, undergo a (chemical) reaction upon irradiation with an energy-carrying activation beam, in particular with electromagnetic radiation. The term "thermo-reactive", as used herein, may in particular mean that the resin composition, in particular some or all of its components, undergo a (chemical) reaction upon supplying the resin composition with thermal energy, such as by heating and/or by irradiation with electromagnetic radiation in the infrared or microwave wavelength regions.

The resin composition may be in particular suitable for printing, for instance in a printing method according to the present invention. Accordingly, the resin composition may be in particular used as an ink (a printing ink), i.e. the resin composition itself may be directly used as an ink. Likewise, the resin composition may be in particular used in an ink (a printing ink), i.e. as a component or an ingredient of an ink together with appropriate one or more further components or ingredients, typically used in an ink.

The resin composition comprises at least two components or ingredients: at least one compound C1 (also referred to as "yne component" or "yne monomer") and at least one compound C2 (also referred to as "thiol component" or "thiol monomer"). As it is evident, the resin composition may contain more than one compound C1 (such as mixtures of different compounds C1) and/or more than one compound C2 (such as mixtures of different compounds C2), as well as further ingredients or components, which will be described below.

The compound C1 has (i) two terminal alkyne functional groups, and (ii) one functional carbonate group.

The term "terminal alkyne functional group", as used herein and as commonly understood by a person skilled in the art, represents a moiety having a carbon-carbon triple bond wherein one of the carbon atoms binds to a hydrogen atom. In other words, a terminal alkyne functional group may be represented by the general formula "-C≡C-H". The terminal alkyne functional group may also be present in protected form, for instance as a silyl (such as trimethylsilyl (TMS)) protected alkyne group and/or as a complex of the carbon-carbon triple bond with for instance dicobalt octacarbonyl. Appropriate deprotection (removal of the protecting group) should then be carried out, as it is well known to a person skilled in the art, prior to the use of the resin composition, for instance in a printing method or in any other intended use thereof where the terminal alkyne functional group is intended to take part in a thiol-yne reaction.

The terms "carbonate", "carbamate" and "ether" as used herein, correspond to the generally accepted meanings thereof. A carbonate may be represented by the general formula "-O(CO)O-", a carbamate may be represented by the general formulas "-(NR)(CO)O-" or "-O(CO)(NR)-" (wherein R may - independently from each other - in particular represent a hydrogen atom, an alkyl group or any other moiety represented by R₁₀ as defined below) and an ether may be represented by the general formula "-O-".

These functional groups enable an appropriate adjustment of physiological properties of a product (such as a polymer or an article comprising or formed the polymer) obtained after the thiol-yne reaction, for instance by a printing method, in a human or animal body, such as when used as a medical or biomedical device. For instance, an ether functional group is hardly hydrolysable under a physiological environment and consequently a product obtained from a resin composition wherein the at least one component C1 comprises an ether functional group is substantially non-biodegradable and is therefore in particular suitable as a dental product, such as a dental prosthesis. On the other hand, a carbamate functional group and in particular a carbonate functional group can be more easily hydrolysed (for instance enzymatically) under a physiological environment and consequently a product obtained from a resin composition wherein the at least one component C1 comprises a carbamate functional group and/or a carbonate functional group is substantially biodegradable and is therefore in particular suitable as an implant, a bone substitute and/or a tissue substitute, which are often intended to gradually degrade and become substituted by natural, physiological material. It might also be advantageous that the product is at least partially biodegradable and at least partially non-biodegradable, for instance if a certain persistent mechanical support is desired, or that a part of the product biodegrades relatively fast whereas a part of the product biodegrades relatively slowly, which might be in particular advantageous when using the product as an implant, a bone substitute and/or a tissue substitute. Thus, also combinations of one carbonate functional group with an ether and/or carbamate functional groups, in particular combinations of one carbonate functional group with an ether functional group, combinations of one carbonate functional group with a carbamate functional group, and combinations of one carbonate functional group with ether and carbamate functional groups, might be appropriate according to specific needs. In addition to one carbonate functional group, the compound C1 may in particular further comprise an ester functional group, which might further provide specific desired physiological properties of the product (such as its degree and rate (speed) of biodegradation under a physiological environment). Other functional groups being more or less hydrolysable/cleavable under physiological conditions may be contained in the compound C1, but also in the compound C2.

The terminal alkyne functional group of the compound C1 is any one selected from the group consisting of propargyl, butynyl and pentynyl. Propargyl may be represented by the general formula "-CH₂-C≡C-H", butynyl may be represented by the general formulas "-CH₂-CH₂-C≡C-H" or "-C(CH₃)H-C≡C-H" and pentynyl may be represented by the general formulas "-CH₂-CH₂-CH₂-C≡C-H", "-C(CH₃)H-CH₂-C≡C-H", "-CH₂-C(CH₃)H-C≡C-H" or "-C(CH₃)₂-C≡C-H". The linear (unbranchend) groups thereof, i.e. "-CH₂-C≡C-H", "-CH₂-CH₂-C≡C-H" and "-CH₂-CH₂-CH₂-C≡C-H" may be preferred.

The present inventors have made the unexpected finding that the larger the carbon atom chain of the terminal alkyne functional group, the higher the reactivity between the terminal alkyne functional group and a thiol group in a thiol-yne reaction, and consequently the faster the resulting crosslinking takes place, which is advantageous for a printing method, in particular a three-dimensional printing method where typically the object to be printed is formed layer by layer requiring the previous layer to be substantially hardened or cured before forming the subsequent layer. Thus, the printing time can be shortened with increasing number of carbon atoms in the terminal alkyne functional group. On the other hand, the present inventors have found that the larger the carbon atom chain of the terminal alkyne functional group, the more flexible the cross-linkages formed, and consequently the mechanical properties of the resulting (polymer) network, in particular its mechanical strength and rigidity, may be impaired, which is not desired when a specific shape and mechanical integrity of the final product or article is an important issue, for instance when used as an implant, a bone substitute and/or a dental product, such as a dental prosthesis.

A terminal alkyne functional group of the compound C1 of any one selected from the group consisting of propargyl, butynyl and pentynyl has proven to be very appropriate for the purposes of the present invention. In particular, a terminal alkyne functional group of the compound C1 of butynyl and/or pentynyl may be preferred in terms of polymerization velocity. In particular, a butynyl group as the at least one terminal alkyne functional group is in particular advantageous in terms of polymerization velocity and mechanical properties of the polymer network formed.

The meaning of the terms "linear", "branched", "saturated", "unsaturated" and "unsubstituted", as used herein, corresponds to the respective well-established meanings thereof, as known to a person skilled in the art. The term "substituted", as used herein, means that one or more, in particular 1, 2, 3, 4, 5, 6, 7, 8, 9 or 10, hydrogen atom(s) of the respective groups are substituted by a substituent. Examples of suitable substituents, include halogen atoms, such as -F, -CI, -Br, -I; -OH, hydroxyalkyl groups (ether), -SH, thioalkyl groups (thioether), =O, carboxyl groups (-COOH) and salts, esters and amides thereof, -NH₂, secondary amine groups, tertiary amine groups, nitrile groups and nitro groups. If two or more substituents are present, they may be the same or different and they may be bound to each other to form a ring. The terms "heteroalkyl group", "heterocycloalkyl group" or "heteroaryl group", respectively, represents an alkyl group, a cycloalkyl group or an aryl group, respectively, wherein one or more, in particular 1, 2, 3, 4, 5, 6, 7, 8, 9 or 10, carbon atoms are replaced by a hetero atom, such as O, N or S, in particular O and/or N. If more than one hetero atom is contained in a group, these hetero atoms may be the same or different.

Suitable examples of the alkyl group include C₁ to C₂₀ alkyl groups, in particular C₂ to C₁₀ alkyl groups, in particular C₃ to C₈ alkyl groups, in particular C4 to C₆ alkyl groups.

Suitable examples of the cycloalkyl group include C₃ to C₂₀ cycloalkyl groups, in particular C4 to C15 cycloalkyl groups, in particular C₅ to C₁₀ cycloalkyl groups, in particular C₆ to C₈ cycloalkyl groups.

Suitable examples of the aryl group include C₆ to C₂₀ aryl groups, in particular C₆ to C₁₆ aryl groups, in particular C₆ to C14 aryl groups, in particular C₆ to C₁₀ aryl groups. In particular, the aryl group may be a phenyl group.

An "aralkyl group", as used herein, denotes a group having an aliphatic and an aromatic moiety, wherein the aliphatic moiety binds to the ether, carbonate or carbamate, respectively, functional group, and wherein the aromatic moiety and/or the aliphatic moiety may optional comprise a hetero atom. In other words, an "aralkyl group" represents an alkyl or cycloalkyl group (or a heteroalkyl or heterocycloalkyl group) having an aryl group (or a heteroaryl group) as a substituent. Suitable aliphatic and aromatic moieties of the aralkyl group correspond to the alkyl, cycloalkyl and aryl groups (or heteroalkyl, heterocycloalkyl and heteroaryl groups, respectively), as defined above.

An "alkaryl group", as used herein, denotes a group having an aliphatic and an aromatic moiety, wherein the aromatic moiety binds to the ether, carbonate or carbamate, respectively, functional group, and wherein the aliphatic moiety and/or the aromatic moiety may optional comprise a hetero atom. In other words, an "alkaryl group" represents an aryl group (or a heteroaryl group) having an alkyl or cycloalkyl group (or a heteroalkyl or heterocycloalkyl group) as a substituent. Suitable aliphatic and aromatic moieties of the alkaryl group correspond to the alkyl, cycloalkyl and aryl groups (or heteroalkyl, heterocycloalkyl and heteroaryl groups, respectively), as defined above.

The at least one compound C1 has two terminal alkyne functional groups. Such compounds C1 may be in particular advantageous when a low viscosity of the resin composition is not required and/or where a particular strong polymeric network with a large variety of cross-linkages is desired.

The compounds C1 as defined herein can be prepared by well-established organic synthesis methods using commercially available starting materials.

The compound C2 has at least two thiol functional groups.

The term "thiol functional group", as used herein and as commonly understood by a person skilled in the art, represents a functional group represented by the general formula "-SH" which may also be present in protected form represented for instance by the general formula "-SZ", wherein "Z" represents a protection group for "-SH", i.e. a thiol protection group.

In an embodiment, at least one of the thiol functional groups comprises a thiol protecting group. In other words, at least one of the thiol functional groups may be present in protected form or at least one of the thiol functional groups may be represented by the general formula "-SZ", wherein "Z" represents a protection group for "-SH", i.e. a thiol protection group. The thiol protection group may be in particular selected from the group consisting of an acyl group, a silyl group and a siloxyl group. In case of an acyl thiol protection group, the thiol functional group in particular represents a thioester. In case of a silyl thiol protection group, the thiol functional group in particular represents a silyl thioether. In case of a siloxyl thiol protection group, the thiol functional group in particular represents a silyl thioester.

Suitable examples for an acyl thiol protection group include formyl and acetyl. Suitable examples for a silyl thiol protection group include trimethylsilyl, triethylsilyl, tert-butyldimethylsilyl, tert-hexylsilyl, trihexylsilyl and tripropylsilyl. Suitable examples for a siloxyl thiol protection group include trimethylsiloxyl, triethylsiloxyl, tert-butyldimethylsiloxyl, tert-hexylsiloxyl, trihexylsiloxyl and tripropylsiloxyl.

Appropriate deprotection (removal of the protecting group) should then be carried out, as it is well known to a person skilled in the art, prior to the use of the resin composition, for instance in a printing method or in any other intended use thereof where the thiol functional group is intended to take part in a thiol-yne reaction. For instance, in case of a silyl thiol protection group (a silyl thioether), deprotection may be carried out by reaction with a photoacid, i.e. a compound that generates an acid upon irradiation with electromagnetic radiation. Likewise, in case of an acyl or a siloxyl thiol protection group (a thioester or a silyl thioester), deprotection may be carried out by reaction with a photobase, i.e. a compound that generates a base upon irradiation with electromagnetic radiation.

Accordingly, the resin composition may further comprise at least one photoacid and/or at least one photobase. In particular, in case of a silyl thiol protection group (a silyl thioether), the resin composition may further comprise at least one photoacid and in case of an acyl or a siloxyl thiol protection group (a thioester or a silylthioester), the resin composition may further comprise at least one photobase.

Suitable examples for a photoacid (photoacid generator, PAG) include
- Ionic photoacid generators, in particular
   ∘ Onium salts, such as onium salts of aryldiazonium, diaryliodonium (e.g. Cyracure UVI-6976 available from The Dow Chemical Company, Esacure 1064 available from Lamberti SpA, QL Cure 211 available from CHANGZHOU TRONLY NEW ELECTRONIC MATERIALS CO.,LTD), triarylsulfonium (e.g. Omnicat 440 available from IGM Resins B.V., Irgacure 250 available from BASF SE, Rhodorsil 207 available from Rhodia), triarylselenonium or triarylphosphonium salts that contain complex halides such as BF₄⁻, SbF₆⁻, AsF₆⁻, B(C₆F₅)₄⁻ or PF₆⁻ as counter ions,
   ∘ Iron arene complexes (e.g. Irgacure 261 available from BASF SE) that contain complex halides such as BF₄⁻, SbF₆⁻, AsF₆⁻, B(C₆F₅)₄⁻ or PF₆⁻ as counter ions,
   ∘ Dialkylphenacyl sulfonium salts that contain complex halides such as BF₄⁻, SbF₆⁻, AsF₆⁻, B(C₆F₅)₄⁻ or PF₆⁻ as counter ions
- Non-ionic photoacid generators, in particular
   ∘ 2-Nitrobenzylester of carboxylic acids
   ∘ 2-Nitrobenzylester of sulfonic acids
   ∘ Sulfones compounds which generate sulfinic acid upon UV irradiation
   ∘ Triarylphosphates
   ∘ N-Hydroxyimide sulfonates (e.g. N-Hydroxy-5-norbornene-2,3-dicarboximide perfluoro-1-butanesulfonate)
   ∘ Sulfonic acid esters of phenol
   ∘ Diazonaphthoquinones
   ∘ Imino sulfonates
   ∘ Trichloromethyl-1,3,5-triazines (e.g. 2-(4-Methoxystyryl)-4,6-bis(trichloromethyl)-1,3,5-triazine)
and/or mixtures of any one of the foregoing.

Suitable examples for a photobase (photobase generator, PBG) include
- Carbamates (e.g. m-nitrophenyl, 3,5-dimethoxybenzyl, 1-methyl-1-(3,5-dimethoxyphenyl)ethyl, α-methylnitropiperonyl, o-nitrobenzyl, 3,4-dimethoxy-6-nitrobenzyl, phenyl(o-nitrophenyl)methyl, 2-(2-nitrophenyl)ethyl, 6-nitroveratryl, 4-methoxyphenacyl, 3'5'-dimethoxybenzoin carbamates)
- o-Acyloximes
- Ammonium salts
- Sulfoamides
- Formamides
- Nifedipines
- Amineimides
- α-Aminoketones
- o-Carbamoyloximes
and/or mixtures of any one of the foregoing.

In an embodiment, the at least one compound C2 may be represented by the following general formula (XIII): 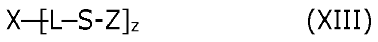 wherein
z represents an integer of from 2 to 1000;
Z represents - independently from each other on each occurrence - hydrogen or a thiol protecting group;
L represents - independently from each other on each occurrence - a single bond or a divalent group selected from the group consisting of a linear or branched, saturated or unsaturated, substituted or unsubstituted alkylene group; a linear or branched, saturated or unsaturated, substituted or unsubstituted heteroalkylene group; a saturated or unsaturated, substituted or unsubstituted cycloalkylene group; a saturated or unsaturated, substituted or unsubstituted heterocycloalkylene group; a substituted or unsubstituted arylene group; a substituted or unsubstituted heteroarylene group; a linear or branched, substituted or unsubstituted aralkylene group; a linear or branched, substituted or unsubstituted alkarylene group; or a silicium containing divalent group; and
X represents a z-valent group selected from the group consisting of a linear or branched, saturated or unsaturated, substituted or unsubstituted alkyl group; a linear or branched, saturated or unsaturated, substituted or unsubstituted heteroalkyl group; a saturated or unsaturated, substituted or unsubstituted cycloalkyl group; a saturated or unsaturated, substituted or unsubstituted heterocycloalkyl group; a substituted or unsubstituted aryl group; a substituted or unsubstituted heteroaryl group; a linear or branched, substituted or unsubstituted aralkyl group; a linear or branched, substituted or unsubstituted alkaryl group; or a silicium containing z-valent group.

In the general formula (XIII), z may in particular represent an integer of from 2 to 1000, with a lower range limit of for instance 2, 3, 4, 5, 6, 7, 8, 9, 10, 11, 12, 13, 14, 15, 16, 17, 18, 19, 20, 25, 30, 35, 40, 45, 50 and/or with an upper range limit of for instance 1000, 900, 800, 750, 700, 650, 600, 550, 500, 450, 400, 350, 300, 275, 250, 225, 200, 190, 180, 170, 160, 150, 140, 130, 125, 120, 115, 110, 105, 100, 95, 90, 85, 80, 75, 70, 65, 60, 55, 50, 45, 40, 38, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, including any single combination of lower and upper range limit values.

The definitions of the terms "linear", "branched", "saturated", "unsaturated", "unsubstituted" and "substituted" may in particular correspond to the above given definitions. Similarly, the definitions and/or suitable examples of the terms "alkylene group", "heteroalkylene group", "cycloalkylene group", "heterocycloalkylene group", "arylene group", "heteroarylene group", "aralkylene group" and "alkarylene group", respectively, may in particular correspond to the above given definitions and/or suitable examples of the terms "alkyl group", "heteroalkyl group", "cycloalkyl group", "heterocycloalkyl group", "aryl group", "heteroaryl group", "aralkyl group" and "alkaryl group", respectively, except that the groups represented by L are divalent (bivalent) groups, as also indicated by their suffix "-ene". Likewise, the definitions and/or suitable examples of the terms "alkyl group", "heteroalkyl group", "cycloalkyl group", "heterocycloalkyl group", "aryl group", "heteroaryl group", "aralkyl group" and "alkaryl group" represented by X may in particular correspond to the above given definitions, except that the groups represented by X are z-valent groups.

The "silicium containing divalent group" and/or the "silicium containing z-valent group" may in particular include groups containing one or more silicium (Si) atoms and optionally further one or more of, for instance, C, O, N, P and/or H atoms. Suitable examples thereof include an Si atom, -[(Si-alkyl)_{z}]- (such as -[(Si-CH₂)_{z}]-), -[(Si-O)z]- and -[(Si-N)_{z}]-.

In an embodiment, Z may represent - independently from each other on each occurrence - hydrogen or a thiol protection group selected from the group consisting of an acyl group, a silyl group and a siloxal group.

In an embodiment, L may - independently from each other on each occurrence - represent a single bond or a divalent group selected from the group consisting of a linear or branched, saturated or unsaturated alkylene group having from 1 to 18 carbon atoms and optionally substituted with one or more hydroxyl groups; - (CO)O-; -O(CO)-; a linear or branched, saturated or unsaturated, divalent alkyl ester group having from 1 to 18 carbon atoms; a linear or branched, saturated or unsaturated, substituted or unsubstituted acylene group having from 1 to 20 carbon atoms; a linear or branched, substituted or unsubstituted alkoxylene group having from 1 to 8 carbon atoms; a saturated or unsaturated, substituted or unsubstituted cycloalkylene group having from 3 to 12 carbon atoms; a substituted or unsubstituted arylene group having from 6 to 16 carbon atoms; or a linear or branched, saturated or unsaturated alkylene group having from 2 to 20 carbon atoms and interspersed with one or more of oxygen, sulfur, a substituted or unsubstituted imine group, -(CO)-, -O(CO)-, -(CO)O-, -O(CO)O-, -(NR₁₀)(CO)O- and/or -O(CO)(NR₁₀)-, with R₁₀ being a hydrogen atom, a linear or branched, saturated or unsaturated, substituted or unsubstituted alkyl group; a linear or branched, saturated or unsaturated, substituted or unsubstituted heteroalkyl group; a saturated or unsaturated, substituted or unsubstituted cycloalkyl group; a saturated or unsaturated, substituted or unsubstituted heterocycloalkyl group; a substituted or unsubstituted aryl group; a substituted or unsubstituted heteroaryl group; a linear or branched, substituted or unsubstituted aralkyl group; or a linear or branched, substituted or unsubstituted alkaryl group.

In an embodiment, X may represent a z-valent group selected from the group consisting of a linear or branched, saturated or unsaturated alkyl group having from 1 to 18 carbon atoms and optionally substituted with one or more hydroxyl groups; a linear or branched, saturated or unsaturated, z-valent alkyl ester group having from 1 to 18 carbon atoms; a linear or branched, saturated or unsaturated, substituted or unsubstituted acyl group having from 1 to 20 carbon atoms; a linear or branched, substituted or unsubstituted alkoxy group having from 1 to 8 carbon atoms; a saturated or unsaturated, substituted or unsubstituted cycloalkyl group having from 3 to 12 carbon atoms; a substituted or unsubstituted arylene group having from 6 to 16 carbon atoms; a linear or branched or cyclic group of any one an Si atom, -[(Si-alkyl)_{z}]- (such as -[(Si-CH₂)_{z}]-), -[(Si-O)_{z}]- or -[(Si-N)_{z}]-, the group being z times substituted by -L-SH; or a linear or branched, saturated or unsaturated alkyl group having from 2 to 20 carbon atoms and interspersed with one or more of oxygen, sulfur, a substituted or unsubstituted imine group, -(CO)-, -O(CO)-, -(CO)O-, -O(CO)O-, -(NR₁₀)(CO)O- and/or -O(CO)(NR₁₀)-, with R₁₀ being as defined above.

In an embodiment, the at least one compound C2 may have at least two thiol functional groups and at least one, in particular at least two, functional group selected from the group consisting of a silane (an Si atom), a siloxane (-Si-O-), a carbonate, a carbamate, an ether and an ester. In particular, the number of thiol functional groups and the number of functional groups selected from the group consisting of a carbonate, a carbamate, an ether and an ester may be the same or different in the at least one compound C2. For instance, the number of thiol functional groups and/or the number of functional groups selected from the group consisting of a carbonate, a carbamate, an ether and an ester may be 2, 3, 4, 5, 6, 7, 8, 9, 10, 11, 12, 13, 14, 15, 16, 17, 18, 19, 20, 25, 30, 35, 40, 45, 50 or more and/or 1000, 900, 800, 750, 700, 650, 600, 550, 500, 450, 400, 350, 300, 275, 250, 225, 200, 190, 180, 170, 160, 150, 140, 130, 125, 120, 115, 110, 105, 100, 95, 90, 85, 80, 75, 70, 65, 60, 55, 50, 45, 40, 38, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3 or less.

Appropriate examples of the at least one compound C2 include for instance the following compounds:

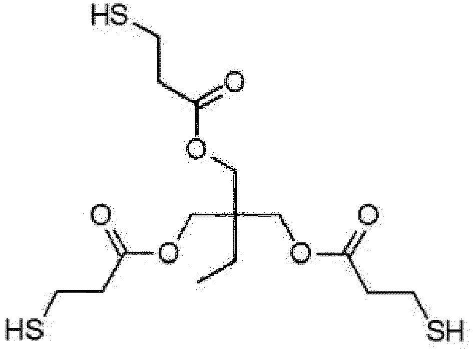

### Trimethylpropane tri(3-mercaptopropionate) (TMPMP)

| | |
|---|---|
| 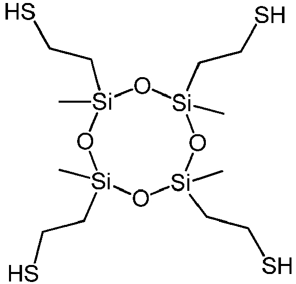 | THIO-1 |
| 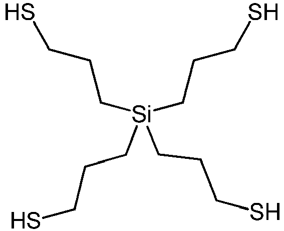 | THIO-2 |
| 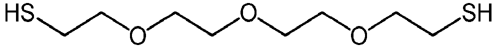 | THIO-3 |
| 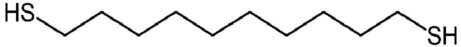 | THIO-4 |
| 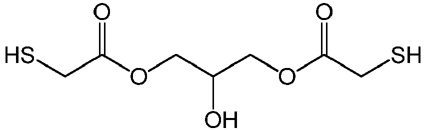 | THIO-5 |
| 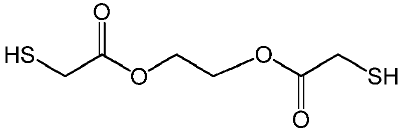 | THIO-6 |
| 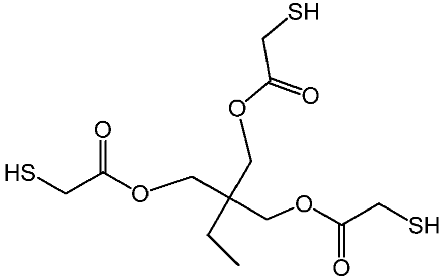 | THIO-7 |
| 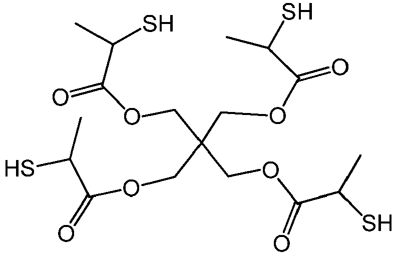 | THIO-8 |

Further appropriate examples of the at least one compound C2 are shown on pages 23 and 24 of WO 2013/052328 A1, the disclosure of which is incorporated herein by reference.

In an embodiment, the ratio of the number of terminal alkyne functional groups to the number of thiol functional groups may be from 1:1.8 to 1:2.25 in the resin composition, in particular from 1:1.85 to 1:2.2, in particular from 1:1.88 to 1:2.15, in particular from 1:1.9 to 1:2.1, in particular from 1:1.95 to 1:2.05, in particular about 1:2. If the ratio of the number of terminal alkyne functional groups to the number of thiol functional groups in the resin composition lies within the above ranges, the number of residual (after completion of the thiol-yne reaction) reactive groups and the number of residual (after completion of the thiol-yne reaction) monomers in the polymer formed can be minimized, which may be advantageous in particular when the polymer (or an article comprising or formed from the monomer) is used in medical or biomedical appliances.

In an embodiment, the ratio of the number of terminal alkyne functional groups to the number of thiol functional groups may be smaller than 1:2, for instance from 1:2.01 to 1:2.3, in particular from 1:2.05 to 1:2.25, from 1:2.1 to 1:2.2. If the ratio of the number of terminal alkyne functional groups to the number of thiol functional groups in the resin composition lies within the above ranges, there may remain some thiol functional groups in the polymer formed after completion of the thiol-yne reaction, in particular on the surface of the polymer or of an article comprising or formed from the polymer. Thereby, a surfacemodified polymer or article may be obtained. In particular, such remaining or residual thiol functional groups may serve as an anchor or docking site (binding site) capable for coupling the polymer or the article with an additive, such as an organic filler (e.g. heparin) and/or an inorganic filler. The binding (coupling) of the remaining or residual thiol functional group with an additive may be carried out for instance by means of a thiol-ene reaction, such as a thiol-Michael addition, typically catalyzed under basic conditions. The binding (coupling) of the remaining or residual thiol functional group with an additive may also involve the formation of disulfide bonds (disulfide bridges), in particular in case of a protein or a peptide to be coupled with the polymer or article. Thereby, the polymer or article can be imparted with additional physiologically beneficial properties, for instance with anticoagulant properties in case of heparin.

The at least one compound C1 may be contained in the resin composition in an amount of from 10 to 90 wt.-%, in particular from 15 to 85 wt.-%, in particular from 20 to 80 wt.-%, in particular from 25 to 75 wt.-%, such as from 20 to 70 wt.-%, from 25 to 65 wt.-%, from 30 to 60 wt.-%, from 35 to 55 wt.-%, from 38 to 52 wt.-% or from 40 to 50 wt.-%.

The at least one compound C2 may be contained in the resin composition in an amount of from 10 to 90 wt.-%, in particular from 15 to 85 wt.-%, in particular from 20 to 80 wt.-%, in particular from 25 to 75 wt.-%, such as from 20 to 70 wt.-%, from 25 to 65 wt.-%, from 30 to 60 wt.-%, from 35 to 55 wt.-%, from 38 to 52 wt.-% or from 40 to 50 wt.-%.

In an embodiment, the resin composition may further comprise at least one initiator selected from the group consisting of a photoinitiator and a thermal initiator. The presence of at least one initiator in the resin composition may facilitate the initiation of the reaction between the yne component and the thiol component in a thiol-yne reaction. Thus, the presence of at least one initiator may increase the polymerization speed (velocity) and/or reduce the curing time. As a consequence, the presence of at least one initiator is in particular advantageous when the resin composition is used as or in an ink for a printing method, in particular a three-dimensional printing method where typically the object to be printed is formed layer by layer requiring the previous layer to be substantially hardened or cured before forming the subsequent layer. In addition by using a photoinitiator and/or a thermal initiator, the thiol-yne reaction can be selectively controlled, in particular in terms of a specific location or area of or within the resin composition where the polymerization is supposed to be initiated/promoted, as desired, for instance by directing an appropriate energy source to the specific location or area, thereby enabling the formation of specific structures with high resolution as a result of a printing method.

The term "photoinitiator", as used herein, in particular denotes a compound which can be activated by an energy-carrying activation beam (such as electromagnetic radiation), for instance upon irradiation therewith. Upon activation by an energy-carrying beam, the photoinitiator may be in particular converted into a radical thereof. Thus, the photoinitiator may be in particular a radical generating photoinitiator.

The photoinitiator may be in particular a type I photoinitiator or a type II photoinitiator.

The photoinitiator may be in particular an ultraviolet-active photoinitiator and/or a visible light-active photoinitiator. In other words, the photoinitiator may be in particular a compound that can be activated by electromagnetic radiation in the ultraviolet wavelength region (such as the wavelength range of from 10 to 380 nm, in particular from 200 to 380 nm) and or by electromagnetic radiation in the wavelength region of visible light (such as the wavelength range of from 380 to 780 nm)

The term "thermal initiator", as used herein, in particular denotes a compound which can be activated by thermal energy, for instance upon directing thermal energy to the compound, such as by heating and/or by irradiation with electromagnetic radiation in the infrared wavelength region (such as the wavelength range of from 780 nm to 1 mm) and/or in the microwave wavelength region (such as the wavelength range of from 1 mm to 300 mm). Upon activation by thermal energy, the thermal initiator may be in particular converted into a radical thereof. Thus, the thermal initiator may be in particular a radical generating thermal initiator.

The photoinitiator and/or the thermal initiator is not particularly limited, as long as it can be activated by electromagnetic radiation and/or thermal energy to thereby initiate the thiol-yne reaction between the yne component and the thiol component in the resin composition.

In an embodiment, the initiator may be at least one compound selected from the group consisting of a quinone compound, camphorquinone, an azide compound, an azo compound, in particular azobisisobutyronitrile (AIBN), a peroxide compound, in particular benzoyl peroxide, a disulfide compound, a bis-imidazole compound, an alkyl halogenide, an alkyl thiocyanate, a phosphinoxide compound, a substituted or unsubstituted thioxanthone, a substituted or unsubstituted benzophenone or mixtures thereof. The term "substituted" may in particular correspond to that, as defined above in, as long as the substituent does not substantially reduce the reactivity of the initiator. As it is well known to a person skilled in the art, these compounds may form radicals and/or moieties having unshared valence electrons or at least one unshared electron pair upon activation by an energy source, such as irradiation with an energy-carrying activation beam and/or thermal energy.

A particularly suitable example of an initiator, in particular of a photoinitiator represents a mixture of 2-hydroxy-2-methyl-1-phenyl propanone and diphenyl 2,4,6-trimethylbenzoyl phosphane oxide, in particular a 50 wt.-%/50 wt.-% mixture thereof. In addition, camphorquinone may be particularly advantageous in view of its high biocompatibility.

Further appropriate examples of initiators, both photoinitiators and thermal initiators, are given on pages 17 to 20 of WO 2013/052328 A1, the disclosure of which is incorporated herein by reference.

The content of the at least one initiator may be in particular from 0.1 to 20 wt.%, such as from 0.2 to 15 wt.-%, in particular from 0.5 to 12.5 wt.-%, in particular from 1 to 10 wt.-%, in particular from 2 to 8 wt.-%, with respect to the total weight of the resin composition.

In an embodiment, the resin composition may further comprise at least one additive selected from the group consisting of a pigment, an inorganic filler, an organic filler and a stabilizer. By the presence of an additive, such as a pigment, an inorganic filler and/or an organic filler, the products obtained by the thiol-yne reaction, for instance the products of a printing method utilizing the resin composition, may be provided with additional specific tailor-made advantageous properties, as desired. The presence of a stabilizer in the resin composition may be helpful to prevent a premature initiation of the thiol-yne reaction between the yne component and the thiol component. Thus, the presence of at least one stabilizer may improve the storage properties of the resin composition.

The pigment may be an organic or an inorganic pigment. The pigment is not particularly limited and any pigment customarily used for printing for example may be used, as long as it does not substantially impair the thiol-yne reaction between the yne component and the thiol component in the resin composition.

Suitable examples of pigments are disclosed for instance in paragraphs [0128] to [0138] of WO 2008/074548, column 14, line 39 to column 15, line 46 of US 6,045,607, pages 12 to 16 of WO 2005/049744 further providing additional references, the disclosures of all of which are incorporated herein by reference. The pigment may be surface-treated so as to improve its dispersibility in the resin composition.

The inorganic filler is not particularly limited, as long as it does not substantially impair the thiol-yne reaction between the yne component and the thiol component in the resin composition. In particular, the inorganic filler may be any one of a calcium carbonate, a calcium phosphate and/or a hydroxyapatite, which are in particular suitable if the product obtained after the thiol-yne reaction, for instance by a printing method, is used as an implant, a bone substitute and/or a dental product, such as a dental prosthesis.

Further appropriate examples of inorganic fillers are disclosed on pages 24 to 27 of WO 2013/052328 A1, the disclosure of which is incorporated herein by reference.

The organic filler is not particularly limited, as long as it does not substantially impair the thiol-yne reaction between the yne component and the thiol component in the resin composition. In particular, the organic filler may be a heparin, a collagen and/or a gelatine, which are in particular suitable if the product obtained after the thiol-yne reaction, for instance by a printing method, is used as an implant, a bone substitute and/or a dental product, such as a dental prosthesis. In addition, the organic filler may comprise physiologically active compounds, such as proteins, peptides, antibodies, drugs, and the like.

Further appropriate examples of organic fillers are disclosed on pages 24 to 27 of WO 2013/052328 A1 and the paragraph bridging pages 7 and 8 of WO 2012/103445 A2, the disclosures of which are incorporated herein by reference.

The stabilizer may be in particular a polymerization inhibitor and may be in particular helpful to prevent a premature initiation and/or propagation of the thiol-yne reaction between the yne component and the thiol component. The stabilizer may be in particular comprise any one or more selected from the group consisting of a phenolic antioxidant, a phosphorous containing compound, a hydroquinone, such as hydroquinone monomethyl ether (HQME), a hydroxyl amine and a sterically hindered amine. In particular, the stabilizer may be a hydroquinone, t-butyl catechol, pyrogallol or an ether thereof, butylhydroxytoluene (BHT, 2,6-di-tert-butyl-4-methylphenol) and/or butylated hydroxyanisole (BHA, 2-tert-butyl-4-hydroxyanisole and/or 3-tert-butyl-4-hydroxyanisole).

Suitable commercial stabilizers are, for example, Sumilizer^{™} GA-80, Sumilizer^{™} GM and Sumilizer^{™} GS available from Sumitomo Chemical Co. Ltd.; Genorad^{™} 16, Genorad^{™}18 and Genorad^{™}20 available from Rahn AG; Irgastab^{™} UV22 available from BASF SE; Additol^{™} S (S100, S110, S120 and S130) available from Cytec Surface Specialities.

Since excessive addition of these stabilizers or polymerization inhibitors may lower the curing speed, it is preferred that the amount capable of preventing polymerization is determined prior to blending. The amount of a polymerization inhibitor is preferably lower than 5 wt.-%, more preferably lower than 3 wt.-% of the total resin composition.

The content of the at least one additive selected from the group consisting of a pigment, an inorganic filler, an organic filler and a stabilizer, in particular its total content, may be in particular from 0.1 to 60 wt.-%, such as from 0.2 to 50 wt.%, in particular from 0.5 to 40 wt.-%, in particular from 1 to 30 wt.-%, in particular from 2 to 20 wt.-%, with respect to the total weight of the resin composition.

Further components or ingredients may be contained in the resin composition.

For example, further monomers other than the above described yne and thiol monomers, for instance vinyl functionalized monomers, may be contained in the resin composition, which may in particular copolymerize with the yne and/or thiol monomers and which may thereby impart further specific characteristics to the (co-)polymer obtained by polymerizing some or all components of the resin composition. Appropriate examples of vinyl functionalized monomers are disclosed for instance in paragraphs [0025] to [0037] of WO 2013/052328 A1, the disclosure of which is incorporated herein by reference.

Moreover, dispersants, wetting agents and/or sequestering agents (chelating agents) may be contained in the resin composition.

In particular, the resin composition may further contain surfactants suitable as dispersants and/or wetting agents, such as anionic surfactants, cationic surfactants, nonionic surfactants and/or ampholytic surfactants.

Suitable examples of anionic surfactants include surfactants comprising carboxylate, sulfate, phosphate and/or sulfonate groups, for instance amino acid derivatives, fatty alcohol ether sulfates, fatty alcohol sulfates, soaps (such as sodium soaps and/or potassium soaps) alkylphenol ethoxylates, fatty alcohol ethoxylates, alkyl sulfates, olefin sulfates and/or alkyl phosphates.

Suitable examples of cationic surfactants include quaternary ammonium or quaternary phosphonium compounds, for instance tetraalkyl ammonium salts, N,N-dialkyl imidazoline compounds, dimethyl distearyl ammonium compounds, N-alkyl pyridine compounds and/or ammonium chlorides.

Suitable examples of nonionic surfactants include ethoxylates, for instance ethoxylated addition products of alcohols, such as polyoxyalkylene polyols, amines, fatty acids, alkyl phenols, ethanol amides, polysiloxanes and/or fatty acid esters, alkyl or alkylphenyl polyglycol ether, such as fatty alcohol polyglycol ether or fatty acid amides, alkyl glycosides, sugar esters, sorbitan esters, polysorbates and/or trialkyl amine oxides; ester and/or amides pf poly(meth)acrylic acids with polyalkylene glycols and/or amino polyalkylene glycols, all of which may be terminated by alkyl groups on one side.

Suitable examples of ampholytic surfactants include amphoteric electrolytes, also called ampholytes, such as amino carboxylic acids and/or betaines.

Suitable commercial examples include Tego Wet and/or Glide from Evonik Industries; Byk 016, 348, UV3500, 9151 from Altana AG; Edaplan 710, 711, 910, 915 from Münzing Chemie GmbH.

Further appropriate examples of additional components or ingredients, in particular of dispersants and surfactants, are disclosed on pages 34 to 37 of WO 2013/087427 A1, the disclosure of which is incorporated herein by reference.

The resin composition may be solid, semi-solid (pasty) or liquid. Since the yne monomer and/or the thiol monomer are typically liquid, the resin composition may be in particular a solution, an emulsion or a dispersion (in particular a solidliquid dispersion, such as a suspension). In particular, the resin composition does not form a hydrogel or any other gel-like form. In particular, the resin composition is not a hydrogel prior to the thiol-yne reaction (prior to polymerization/curing) nor after the thiol-yne reaction (after polymerization/ curing). Rather, after the thiol-yne reaction (after polymerization/curing) a polymer, in particular a solid or semi-solid (pasty) polymer is formed.

The resin composition may be in particular substantially solvent-free, such as substantially water-free. The term "substantially solvent-free", as used herein, may in particular denote that the resin composition comprises not more than 15 wt.-% of a solvent, in particular not more than 10 wt.-%, in particular not more than 5 wt.-%, in particular not more than 2 wt.-%, in particular not more than 1 wt.-%.

Accordingly, the resin composition may in particular contain substantially no solvent, such as a polar solvent or an apolar solvent, for instance water, an alcoholic solvent (such as methanol, ethanol, glycol, 1-propanol, 2-propanol (IPA), propylene glycol, 1-butanol, 2-butanol, isobutyl alcohol, butylene glycol, and the like), an ether solvent (such as dimethyl ether, diethyl ether, tert-butyl methyl ether, 1,4-dioxane, tetrahydrofuran (THF), and the like), an ester solvent (such as ethyl acetate, and the like), a carbonate solvent (such as dimethyl carbonate, diethyl carbonate, and the like), a halogenated alkane solvent (such as dichloromethane, trichloromethane, tetrachloromethane, 1,2-dichloroethane, and the like), a nitrile solvent (such as acetonitrile, and the like), an aldehyde or ketone solvent (such as acetone, and the like), an amide solvent (such as dimethylformamide (DMF), and the like), a sulfoxide solvent (such as dimethylsulfoxide (DMSO), and the like), an acid solvent (such as formic acid, acetic acid, and the like), a hydrocarbon solvent (such as pentane, cyclopentane, hexane, cyclohexane, heptane, octane, and the like), or an aromatic solvent (such as benzene, toluene, and the like).

In an embodiment, the at least one compound C1 and the at least one compound C2 may form one (single) compound. In other words, the resin composition may comprise (or consist of) at least one compound C3 having
(i) two terminal alkyne functional groups selected from the group consisting of propargyl, butynyl and pentynyl,
(ii) one functional carbonate group, and
(iii) at least two thiol functional groups.

The two terminal alkyne functional groups, the at least one functional carbonate group, and/or the at least two thiol functional groups of the compound C3 may in particular be those, as illustrated in the foregoing. In addition, the resin composition comprising at least one compound C3 may further comprise further ingredients or components, as described in detail above, in particular the at least one initiator selected from the group consisting of a photoinitiator and a thermal initiator, the photoacid, the photobase, the at least one additive selected from the group consisting of a pigment, an inorganic filler, an organic filler and a stabilizer, the further monomers other than the yne and thiol monomers, surfactants, dispersants, wetting agents and/or sequestering agents.

By comprising at least one compound C3, the resin composition may show particularly advantageous properties, such as a high reaction (polymerization) speed (high curing velocity), a particularly low content of residual monomers, a particularly low shrinkage of the resulting polymer, excellent mechanical properties (such as elastic modulus) of the resulting polymer, and the like.

In a second aspect, the present invention relates to the use of the resin composition according to the present invention as or in an ink, which may be for instance suitable in a printing method, as described in further detail below. In particular, the resin composition may be used as an ink (a printing ink), i.e. the resin composition itself may be directly used as an ink. Alternatively, the resin composition may be used in an ink (a printing ink), i.e. as a component or an ingredient of an ink together with appropriate one or more further components or ingredients, typically used in an ink.

In a third aspect, the present invention relates to a kit comprising:
at least one compound C1 having
   (i) two terminal alkyne functional groups selected from the group consisting of propargyl, butynyl and pentynyl, and
   (ii) one carbonate functional group; and
at least one compound C2 having at least two thiol functional groups.

The at least one compound C1 as well as the at least one compound C2 may in particular be those as defined in detail above with regard to the resin composition according to the present invention.

The components C1 and C2 may be in particular provided in a spatially separated manner in the kit, in particular the kit-of-parts. In particular, the components C1 and C2 may be provided in separate compartments of the kit. This might be advantageous if the at least one component C1 and/or at least one component C2 are reactive to such an extent that it/they tends/tend to (prematurely) react with the other component/with each other, even when stored in the dark (such as when packaged by means of a light nontransparent material) and/or at low temperature (such as at a temperature of not more than 10 °C, in particular not more than 5 °C, such as not more than 0 °C).

Further ingredients or components, as described in detail above with regard to the resin composition according to the present invention, in particular the at least one initiator selected from the group consisting of a photoinitiator and a thermal initiator, the photoacid, the photobase, the at least one additive selected from the group consisting of a pigment, an inorganic filler, an organic filler and a stabilizer, the further monomers other than the yne and thiol monomers, surfactants, dispersants, wetting agents and/or sequestering agents, may be contained independently from each other in any one of the compartment of compound C1 and/or the compartment of compound C2 as well as in any additional compartment of the kit.

In particular, the kit may be a two-, three-, four- or multi-component system, such as a 2K system, a 3K system, a 4K system and the like.

Prior to use, for instance in a printing method, the components contained in separate compartments of the kit are mixed. The mixing may be carried out manually or (semi-)automatically by an appropriate device or dispenser. The components contained in separate compartments of the kit may be in particular mixed not more than 48 hours, not more than 24 hours, not more than 12 hours, not more than 6 hours, not more than 4 hours, not more than 3 hours, not more than 2 hours, not more than 90 minutes, not more than 60 minutes, not more than 45 minutes, not more than 30 minutes, not more than 25 minutes, not more than 20 minutes, not more than 15 minutes, not more than 10 minutes, not more than 7.5 minutes, not more than 5 minutes, not more than 4 minutes, not more than 3 minutes, not more than 2 minutes, not more than 90 seconds, not more than 60 seconds, not more than 45 seconds, not more than 30 seconds, not more than 25 seconds, not more than 20 seconds, not more than 15 seconds, not more than 10 seconds, not more than 7.5 seconds, not more than 5 seconds, not more than 4 seconds, not more than 3 seconds, not more than 2 seconds, not more than 1 second, prior to use, for instance in a printing method.

In a fourth aspect, the present invention relates to the use of the kit according to the present invention for preparing a resin composition, in particular the resin composition according to the present invention, suitable for use as or in an ink, which may be for instance suitable in a printing method, as described in further detail below. For preparing the resin composition, the components contained in separate compartments of the kit may be mixed. The mixing may be carried out manually or (semi-)automatically by an appropriate device or dispenser.

In a fifth aspect, the present invention relates to a printing method comprising the steps of
providing the resin composition according to the present invention, wherein the resin composition further comprises at least one initiator; and
directing an energy source to at least a part of the resin composition so as to cause polymerization of the at least a part of the resin composition and so as to obtain a polymer.

In an embodiment, the printing method may be a two-dimensional printing method and the resin composition may be provided on a substrate.

The term "two-dimensional printing method", as used herein, in particular denotes that the product of the printing method extends in two directions (for example, length and width), but not in a third direction (for example, height). Thus, the product of a two-dimensional printing method may be substantially flat or sheet-like.

The two-dimensional printing method may be in particular an ink-jet printing method or a laser printing method.

The substrate may be flexible, but it may also possess a certain rigidity or may be rigid, as desired. The material of the substrate is not particularly limited and various suitable materials are known to a person skilled in the art. In particular, the substrate may be paper or any other cellulose-based material suitable for printing. Further appropriate substrate materials include polyester, polysulfones, polyarylates, polyacrylates, polycyclic olefins, polyimides, glass or combinations or composites thereof. Merely by way of example, further substrate materials include polyester, such as polyethylene terephthalate (PET), polyethylene naphthalate (PEN), polycarbonate (PC); polysulfones, such as polyethersulfone (PES); polyarylates (PAR); polycyclic olefins (PCO); polyimides (PI); polyolefins, such as polyethylene (PE), polypropylene (PP); vinyl polymers, such as polystyrene (PS), polyvinyl chloride (PVC), polymethyl methacrylate (PMMA); polyamides; polyethers; polyketones, such as aromatic polyetherketones (e.g. PEEK); polysulfides (e.g. PPS); fluoropolymers, such as polyvinylidene fluoride (PVDF), polytetrafluoroethylene (such as PTFE), fluorinated ethylene propylene (FEP); liquid crystal polymers; polyepoxides; polyurethanes; polysiloxanes (e.g. PDMS); biopolymers or combinations, copolymers, composites and/or blends thereof, in particular composites or laminates thereof with paper or any other cellulose-based material suitable for printing.

In an embodiment, the printing method may be a three-dimensional printing method.

The term "three-dimensional printing method", as used herein, in particular denotes that the product of the printing method extends in three directions (for example, length, width and height). Thus, the product of a three-dimensional printing method may be in particular a three-dimensional object.

The three-dimensional printing method may be in particular any one selected from the group consisting of stereolithography (SLA), two-photon absorption (TPA) polymerization, digital light processing (DLP), solid ground curing (SGC) and multi jet modeling (MJM). In view of its high resolution, stereolithography (SLA) may be preferred.

In the step of providing the resin composition, the resin composition further comprises an initiator, in particular a photoinitiator and/or a thermal initiator. The initiator may be in particular that as defined in detail above with regard to the resin composition according to the present invention.

In the step of directing an energy source to at least a part of the resin composition (curing step), a thiol-yne reaction may be caused at the at least a part of the resin composition, to which the energy source has been directed, with the result that the yne component and the thiol components undergo a crosslinking (polymerization) reaction at that part and consequently a polymer is formed at that part of the resin composition, to which the energy source has been directed.

In particular, the energy source may be directed to the at least a part of the resin composition in a controlled manner, in particular controlled by a computer system, so as to form a desired pattern or structure of the resulting polymer.

The term "at least a part of the resin composition" may in particular mean that not 100 % of the resin composition is exposed to the energy source. In particular, the term "at least a part of the resin composition" may mean that at least 5 %, in particular at least 10 %, in particular at least 15 %, in particular at least 20 %, in particular at least 25 %, in particular at least 30 %, in particular at least 35 %, in particular at least 40 %, in particular at least 45 %, in particular at least 50 %, in particular at least 55 %, in particular at least 60 %, in particular at least 65 %, in particular at least 70 %, in particular at least 75 %, in particular at least 80 % of the resin composition is exposed to the energy source and it may mean that in particular not more than 95 %, in particular not more than 90 %, in particular not more than 85 %, in particular not more than 80 %, in particular not more than 75 %, in particular not more than 70 %, in particular not more than 65 %, in particular not more than 60 %, in particular not more than 55 %, in particular not more than 50 %, in particular not more than 45 %, in particular not more than 40 %, in particular not more than 35 %, in particular not more than 30 %, in particular not more than 25 %, in particular not more than 20 % of the resin composition is exposed to the energy source.

It might be advantageous to carry out the step of directing an energy source to at least a part of the resin composition under an inert gas atmosphere (such as under a N₂, CO₂, or a noble gas, in particular Ar, atmosphere), while it is also possible to carry out this step under ambient gas atmosphere, such as air, or even under (substantially) pure oxygen in view of the intensity of the thiol-yne reaction against oxygen.

The duration of the step of directing an energy source to at least a part of the resin composition is not particularly limited, and may be appropriately selected by a person skilled in the art, depending in particular on the type of the printing method and the components of the resin composition (in particular their reactivity). For instance, suitable times (durations) for directing an energy source may be from 1 ms to 1 h, in particular from 1 s to 1 min.

The intensity of directing an energy source is not particularly limited, and may be appropriately selected by a person skilled in the art, depending in particular on the type of the printing method, the components of the resin composition and the duration of the step. For instance, the intensity may be from 0.01 to 20 W/cm², in particular from 0.1 to 5 W/cm².

In an embodiment, the step of directing an energy source to at least a part of the resin composition, may comprise or may be followed by a step of post-curing, wherein the initially formed polymer is further or again provided with energy (from an energy source). For instance, in case of stereolithography, the (initially) formed polymer may be further or again irradiated with ultraviolet radiation in a post-curing step.

In an embodiment, the at least one initiator may comprise at least one photoinitiator and the step of directing an energy source to at least a part of the resin composition may comprise irradiating the at least a part of the resin composition with an energy-carrying activation beam.

The energy-carrying activation beam may in particular comprise electromagnetic radiation (in particular actinic radiation).

In particular, the energy-carrying activation beam may be at least one selected from the group consisting of ultraviolet radiation (such as having a wavelength of from 10 to 380 nm, in particular from 200 to 380 nm, in particular from 250 to 380 nm), visible light radiation (such as having a wavelength of from 380 to 780 nm), infrared radiation (such as having a wavelength of from 780 nm to 1 mm, in particular near-infrared radiation having a wavelength of from 780 nm to 1.4 µm), microwave radiation (such as having a wavelength of from 1 to 300 mm), gamma radiation (such as having a wavelength of from 0.1 to 5 pm), X-ray beam (such as having a wavelength of from 1 pm to 10 nm) or electron beam (such as beta radiation).

In an embodiment, the at least one initiator may comprise at least one thermal initiator and the step of directing an energy source to at least a part of the resin composition comprises directing thermal energy to the at least a part of the resin composition.

The thermal energy may be directed to the at least a part of the resin composition by specifically heating that part of the resin composition, such as by contact heating. The thermal energy may also be directed to the at least a part of the resin composition by irradiating the at least a part of the resin composition with an energy-carrying activation beam that causes a (local) increase in temperature on and/or in the irradiated part of the resin composition, such as infrared radiation and/or microwave radiation.

In particular, the printing method may be a solvent-free printing method. In particular, those solvents as described in detail above with regard to the resin composition according to the present invention are preferably not used in the printing method.

In a sixth aspect, the present invention relates to a polymer obtainable by the printing method according to the present invention. The polymer may not only be defined by the components of the resin composition, but also it may be defined by the specific pattern and/or (three-dimensional) structure that it has obtained by the printing method. The chemical structure of the polymer may in particular depend on the components of the resin composition, but also on the specific conditions of the printing method that may influence the degree of crosslinking within the polymer, whereas the geometrical structure of the polymer may in particular depend on the specific conditions of the printing method, such as the specific pattern and/or (three-dimensional) structure imparted to the polymer by the printing method, but also on the components of the resin composition, in particular as regards the flexibility/rigidity of the polymer.

In a seventh aspect, the present invention relates to an article comprising or formed from the polymer according to the present invention. The article may consist of the polymer with or without any further modifications, such as reshaping, or the article may comprise the polymer in addition to further components or ingredients, as desired according to specific purposes. The article may also be formed from the polymer.

In an embodiment, the article may be a medical device or a biomedical device. The medical and/or biomedical device may be in particular selected from the group consisting of an implant, a bone substitute, a tissue substitute and a dental product.

Since a medical and/or biomedical device is typically exposed to a human or animal body upon use, the article should preferably be biocompatible. In particular, the article should not substantially disturb/impart the physiological functions or properties of that part of a human or animal body to be in contact with the article. In addition, the article should not release any harmful compounds or any otherwise detrimental compounds to that part of a human or animal body to be in contact with the article. Since the polymer and consequently also the article is obtained as a result of a thiol-yne reaction, the polymer has a very low residual monomer content, a high chemical stability and a low shrinkage so that the polymer as well as the article is not only highly biocompatible, but is also in particular suitable as a medical and/or biomedical device.

In an embodiment, the article (as well as the polymer) may be substantially biodegradable.

The term "substantially" as used herein in particular denotes at least 50%, in particular at least 60%, in particular at least 70%, in particular at least 75%, in particular at least 80%, in particular at least 85%, in particular at least 90%, in particular at least 92.5%, in particular at least 95%, in particular at least 96%, in particular at least 97%, in particular at least 98%, in particular at least 99%, in particular up to 100%, unless specifically stated otherwise.

As explained above, a carbamate functional group and in particular a carbonate functional group can be relatively easily hydrolysed (for instance enzymatically) under a physiological environment and consequently a product (such as the polymer and/or the article) obtained from a resin composition wherein the at least one component C1 comprises a carbamate functional group and/or a carbonate functional group is substantially biodegradable and is therefore in particular suitable as an implant, a bone substitute and/or a tissue substitute, which are often intended to gradually degrade and become substituted by natural, physiological material. Thus, a substantially biodegradable article (or polymer) may in particular be derived from a resin composition comprising at least one compound C1 comprising a carbamate functional group and/or a carbonate functional group. In addition, a substantially biodegradable article (or polymer) may in particular be suitable as an implant, a bone substitute and/or a tissue substitute.

In an alternative embodiment, the article (as well as the polymer) may be substantially non-biodegradable.

As explained above, an ether functional group is hardly hydrolysable under a physiological environment and consequently a product (such as the polymer and/or the article) obtained from a resin composition wherein the at least one component C1 comprises an ether functional group is substantially non-biodegradable and is therefore in particular suitable as a dental product, such as a dental prosthesis. Thus, a substantially non-biodegradable article (or polymer) may in particular be derived from a resin composition comprising at least one compound C1 comprising an ether functional group. In addition, a substantially non-biodegradable article (or polymer) may in particular be suitable as a dental product, such as a dental prosthesis.

It might also be advantageous that the product (such as the polymer and/or the article) is at least partially biodegradable and at least partially non-biodegradable, for instance if a certain persistent mechanical support is desired, or that a part of the product biodegrades relatively fast whereas a part of the product biodegrades relatively slowly, which might be in particular advantageous when using the product as an implant, a bone substitute and/or a tissue substitute. Such products may in particular be derived from a resin composition comprising combinations of ether, carbamate and/or carbonate functional groups, in particular combinations of ether and carbamate functional groups, combinations of ether and carbonate functional groups, combinations of carbamate and carbonate functional groups, and combinations of ether, carbamate and carbonate functional groups.

In an eighth aspect, the present invention relates to the use of the polymer or of the article according to the present invention in a medical or biomedical application.

In particular, the medical application comprises any one selected from the group consisting of an implantation, a bone substitution or replacement, a tissue substitution or replacement, and a dental application.

### Examples

### Example 1

The maximum reaction speed of various monomers was evaluated by means of Photo Differential Scanning Calorimetry (Photo-DSC). 8 mg of the respective test mixtures consisting of yne monomer (as shown in the following table 1) and thiol monomer (trimethylpropane tri(3-mercaptopropionate), TMPMP) as well as 5 wt.% of a photoinitiator mixture (2-hydroxy-2-methyl-1-phenyl propanone/diphenyl 2,4,6-trimethylbenzoyl phosphane oxide, 50 wt.-%/50 wt.-%) were provided. As comparative test mixtures, acrylate and methacrylate without thiol monomer, but together with 5 wt.-% of the same photoinitiator mixture were provided. The test mixtures were placed in a crucible of aluminium and irradiated with an Omnicure^{®} lamp at an intensity of 1 W/cm² at 50 °C under a nitrogen gas atmosphere to thereby cure the test mixtures.

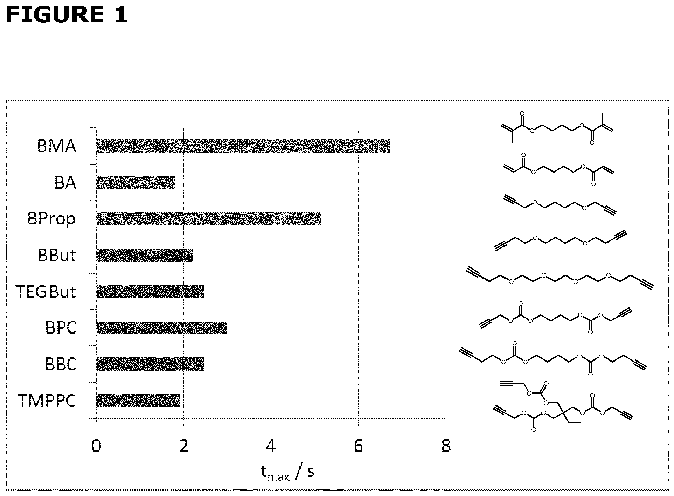

Table 1 shows the results of the maximum reaction speed evaluation of the monomers tested. The times (tₘₐₓ) for reaching the maximum reaction enthalpy indicate the reactivity of the photo-reactive systems. Figure 1 is a graphical illustration of the test results (tₘₐₓ) further showing the chemical structural formulas of the tested alkyne monomers and comparative (meth)acryl monomers.

**[Table 1]**

| **Monomer tested** | **tmax [s]** |
|---|---|
| 1,4-butanediol dimethacrylate (BMA)^{∗} | 6.72 |
| 1,4-butanediol diacrylate (BA)^{∗} | 1.80 |
| 1,4-butanediol dipropargyl ether (BProp)^{∗∗} | 5.12 |
| 1,4-butanediol dipropargyl carbonate (BPC)^{∗∗} | 3.00 |
| 1,4-triethyleneglycol dibut-1-ynyl ether (TEGBut)^{∗∗} | 2.46 |
| 1,4-butanediol dibut-1-ynyl ether (BBut)^{∗∗} | 2.22 |
| 1,4-butanediol dibut-1-ynyl carbonate (BBC)^{∗∗} | 2.46 |
| Trimethylolpropane tripropargyl carbonate (TMPPC)^{∗∗} | 1.92 |

| | |
|---|---|
| ^{∗} Comparative compounds ^{∗∗} not within the scope of the present invention | |

As a result, the reaction speeds of the tested monomers were slightly lower than the reaction speed of a comparable acrylate, but significantly higher than the reaction speed of a comparable methacrylate.

### Example 2

The reaction kinetics of the monomers listed in Table 1 were further evaluated by means of Real-Rime-FTIR spectroscopy. 2 µl of the liquid to be tested (alkyne monomer, TMPMP and 5 wt.-% photoinitiator; acrylate or methacrylate, respectively, and 5 wt.-% photoinitiator) were placed between two discs of CaF₂ and positioned within the optical path. The samples were irradiated by means of Omnicure^{®} S1000 (at 40% of its maximum power) from a distance of 9 cm. Two measurements per second were recorded. The total duration of measurement was 120 seconds.

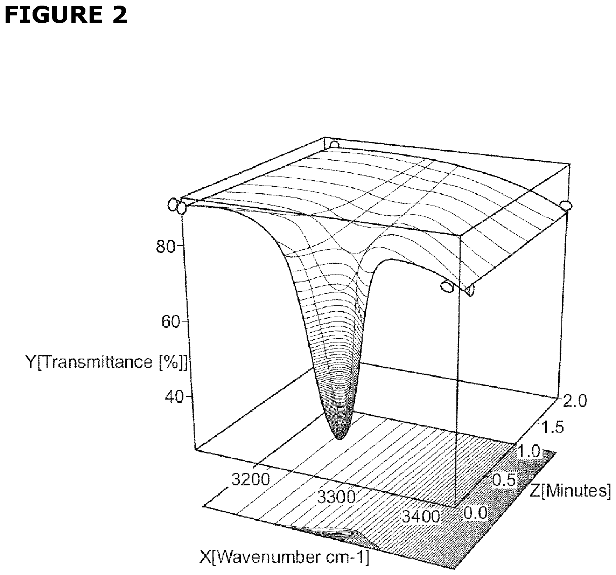

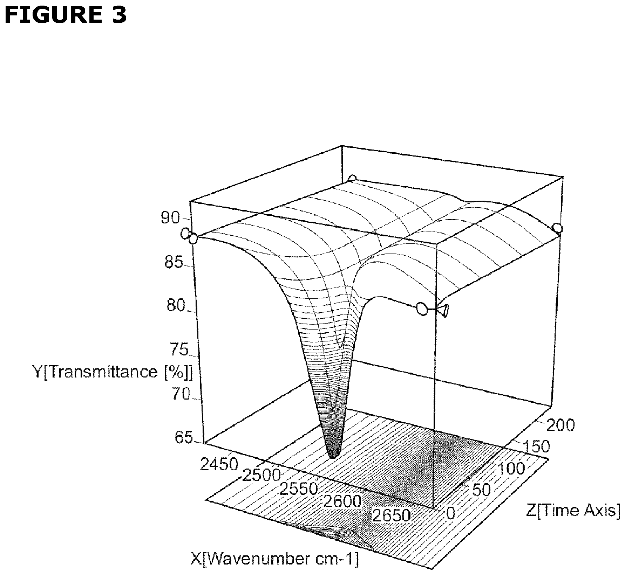

Figures 2 and 3 are graphical three-dimensional illustrations of the temporal decrease of the alkyne peak (at 3280 cm⁻¹) (Figure 2) and of the thiol peak (at 2565 cm⁻¹) (Figure 3) of TMPMP in a thiol-yne reaction with triethyleneglycol dibut-1-ynyl ether. As it is evident from these illustrations, a substantially complete conversion is achieved for both species.

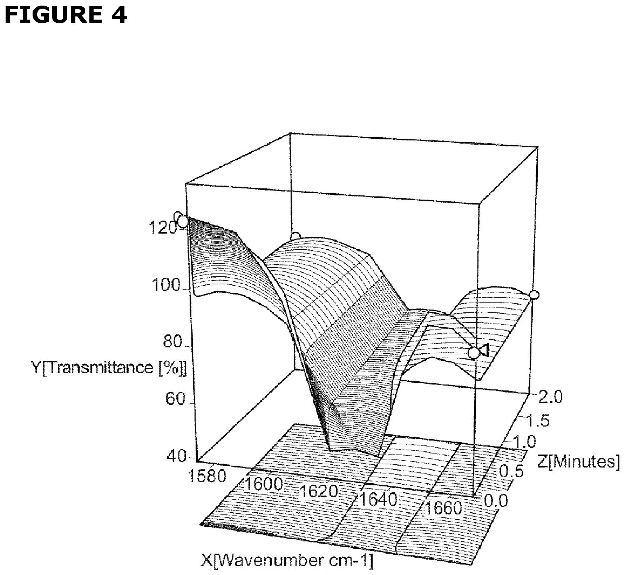

Figure 4 is a graphical three-dimensional illustration of the temporal decrease of the alkene band (at 1640 cm⁻¹) of 1,4-butanediol diacrylate. As it is evident from this illustration, an incomplete conversion can only be achieved.

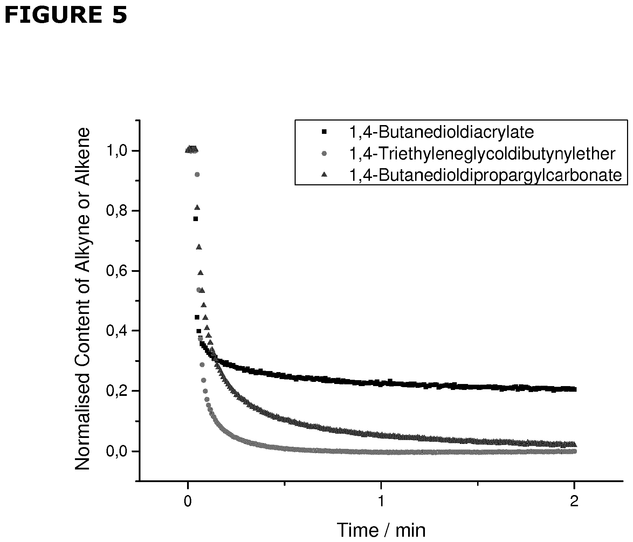

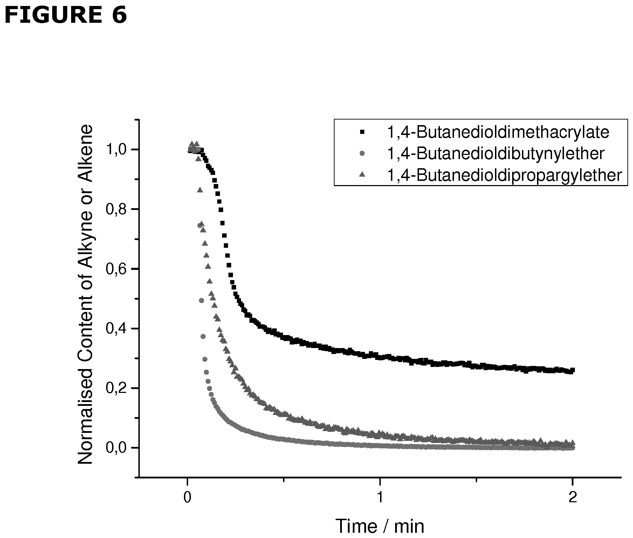

For illustrating the conversion kinetics in further detail, the peak areas of the alkyne band (at 3280 cm⁻¹) and of the alkene band (at 1640 cm⁻¹) of the (meth)acrylates were integrated by means of the software OPUS from Bruker Corporation. The thus obtained, normalised values are shown in Figures 5 and 6.

In addition, the content of alkyne and alkene, respectively, was determined after a reaction time of 2 min, which corresponds to the content of residual monomers in %. The results are shown in the following Table 2.

**[Table 2]**

| **Monomer** | **Residual monomer content** [**%**] |
|---|---|
| 1,4-butanediol dimethacrylate^{∗} | 26 |
| 1,4-butanediol diacrylate^{∗} | 21 |
| 1,4-butanediol dipropargyl ether^{∗∗} | 2 |
| 1,4-butanediol dipropargyl carbonate^{∗∗} | 2 |
| 1,4-butanediol dibut-1-ynyl ether^{∗∗} | < 1 |
| 1,4-triethyleneglycol dibut-1-ynyl ether^{∗∗} | < 1 |

| | |
|---|---|
| ^{∗} Comparative compounds ^{∗∗} not within the scope of the present invention | |

As it is evident from the results shown in Figures 5 and 6, a substantial complete conversion of the alkynes after the thiol-yne reaction was observed, which is in particular advantageous in that it results in a low residual monomer content of 2% or less, as shown in Table 2. In contrast, in case of 1,4-butanediol diacrylate a residual monomer content of 21% was observed even after 2 minutes of irradiation and in case of 1,4-butanediol dimethacrylate a residual monomer content of even 26% was observed. It has thereby been proven that the step growth mechanism of the thiol-yne reaction having a delayed gelling point is far advantageous over the chain growth mechanism of the (meth)acrylates. Due to the substantially lower residual monomer content of polymers obtained by the thiol-yne reaction, less migration of monomers is likely to occur when used as a medical or biomedical device, which renders these polymers particularly suitable for use as or in a medical or biomedical device.

### Example 3 (Biocompatibility)

The cytotoxicity of various monomers was determined by a certified laboratory by means of L929 mice fibroblast cells. As a (toxic) positive control, Triton X 100 was added to L929 cells with a final concentration of 1 % (v/v). As a (non-toxic) negative control, a cell culture medium was used.

After completion of the incubation, the residual protein content of the cells was determined. A reduction of the protein content from L929 cells by more than 30%, relative to the negative control, was evaluated as cytotoxic (which corresponds to the toxicological concentration limit). From this, the half maximal effective concentration (EC₅₀), at which 50% of the cells are damaged, can be calculated.

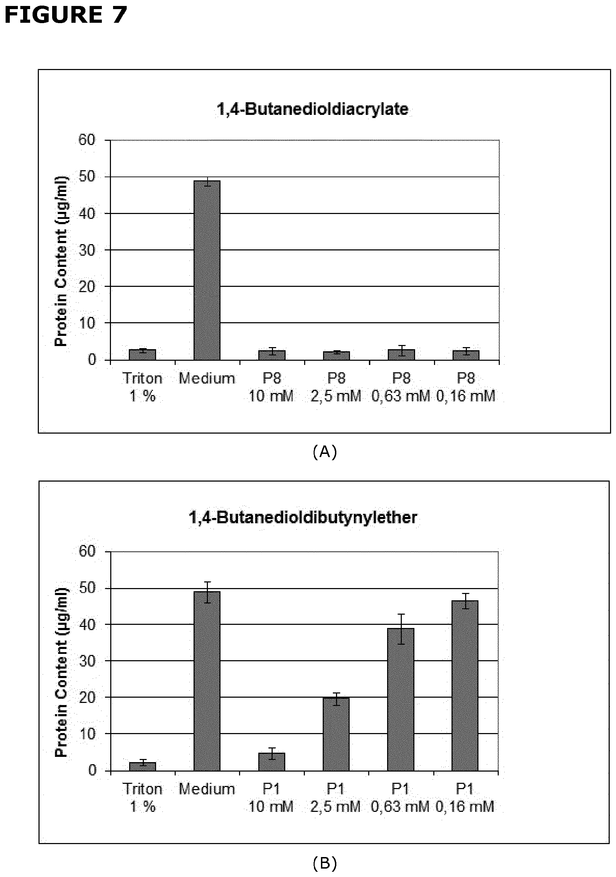

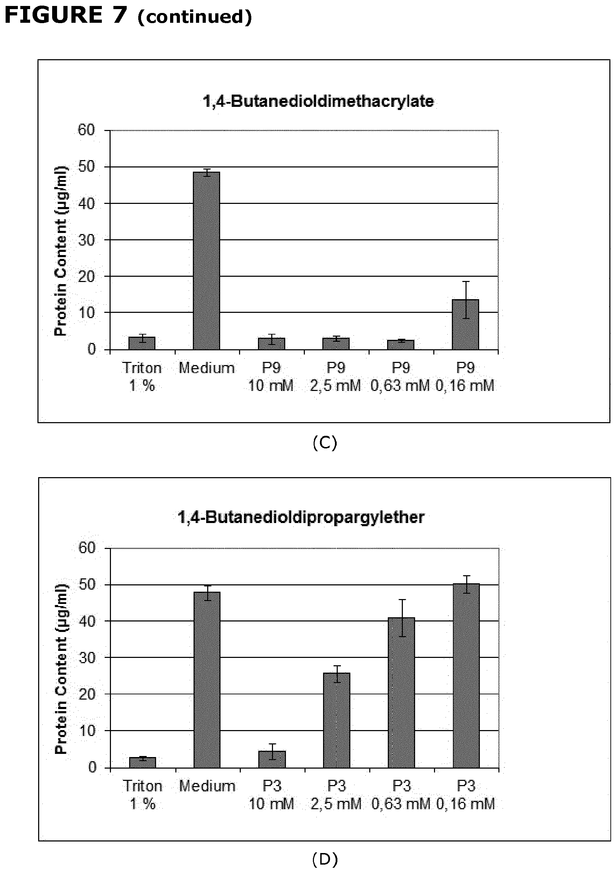

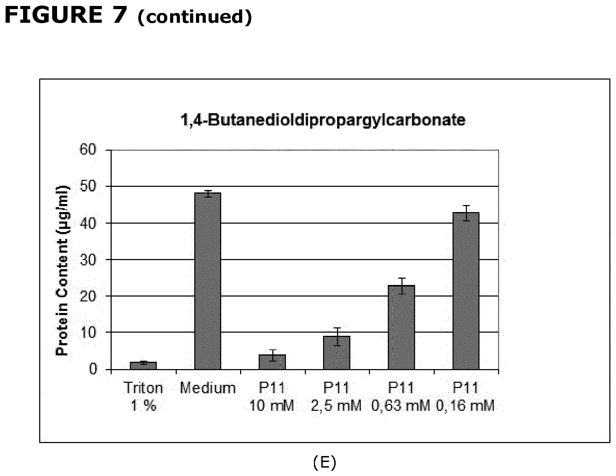

The results obtained from various monomers are shown in Figure 7 (A) to (E).

Table 3 shows the determined concentration limits as well as the EC₅₀ values of the monomers tested.

**[Table 3]**

| **Monomer** | **Concentration limit [mM]** | **EC₅₀ [mM]** |
|---|---|---|
| 1,4-butanediol dimethacrylate^{∗} | < 0.16 | - |
| 1,4-butanediol diacrylate^{∗} | < 0.16 | - |
| 1,4-butanediol dipropargyl ether^{∗∗} | > 1.6 | 3.0 |
| 1,4-butanediol dibut-1-ynyl ether^{∗∗} | > 1.1 | 2.0 |
| 1,4-butanediol dipropargyl carbonate^{∗∗} | > 0.3 | 0.55 |

| | | |
|---|---|---|
| ^{∗} Comparative compounds ^{∗∗} not within the scope of the present invention | | |

As it is evident from the results of the cytotoxicological evaluation, all tested alkyne monomers show a significantly lower cytotoxicity than their respective (meth)acrylate analogues. 1,4-Butanediol diacrylate as well as 1,4-butanediol dimethacrylate have proven to be particularly cytotoxic. An EC₅₀ value of these compounds could not be calculated because the toxic concentration was beneath the detection limit of the measurement method. Among the tested alkyne monomers, in particular 1,4-butanediol dipropargyl ether as well as 1,4-butanediol dibut-1-ynyl ether revealed an excellently low cytotoxicity.

### Example 4 (Measurement of viscosity)

For measuring the viscosity, an MC 200 cone-and-plate viscosimeter from Anton Paar GmbH (cone unit MK 22/50 mm, 1°) was used. First, the shear rate was varied with a stepped slope and subsequently the viscosity was determined at a constant shear rate (300 s⁻¹) every 30 seconds. The measurement was carried out at 25 °C.

The results of the viscosity measurements are shown in Table 4

**[Table 4]**

| **Monomer** | **Viscosity at 25 °C [mPa^{∗}s]** |
|---|---|
| 1,4-butanediol dimethacrylate^{∗} | 4.6 |
| 1,4-butanediol diacrylate^{∗} | 4.3 |
| 1,4-butanediol dipropargyl ether^{∗∗} | 4.7 |
| 1,4-butanediol dipropargyl carbonate^{∗∗} | 44.2 |
| 1,4-butanediol dibut-1-ynyl ether^{∗∗} | 6.1 |
| 1,4-triethyleneglycol dibut-1-ynyl ether^{∗∗} | 17.2 |

| | |
|---|---|
| ^{∗} Comparative compounds ^{∗∗} not within the scope of the present invention | |

The viscosity values determined from 1,4-butanediol dipropargyl ether as well as from 1,4-butanediol dibut-1-ynyl ether show relatively low values, comparable to those of the (meth)acrylates. 1,4-Butanediol dipropargyl carbonate revealed a significantly higher viscosity.

## Claims

1. A resin composition comprising:
at least one compound C1 having
(i) two terminal alkyne functional groups selected from the group consisting of propargyl, butynyl and pentynyl, and
(ii) one functional carbonate group; and
at least one compound C2 having at least two thiol functional groups.

2. The resin composition according to claim 1, wherein at least one of the thiol functional groups comprises a thiol protecting group, in particular wherein the thiol protection group is selected from the group consisting of an acyl group, a silyl group and a siloxyl group, and/or wherein the resin composition further comprises at least one photoacid and/or at least one photobase.

3. The resin composition according to any one of the preceding claims, wherein the at least one compound C2 is represented by the following general formula (XIII): 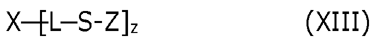 wherein
z represents an integer of from 2 to 1000;
Z represents - independently from each other on each occurrence - hydrogen or a thiol protecting group;
L represents - independently from each other on each occurrence - a single bond or a divalent group selected from the group consisting of a linear or branched, saturated or unsaturated, substituted or unsubstituted alkylene group; a linear or branched, saturated or unsaturated, substituted or unsubstituted heteroalkylene group; a saturated or unsaturated, substituted or unsubstituted cycloalkylene group; a saturated or unsaturated, substituted or unsubstituted heterocycloalkylene group; a substituted or unsubstituted arylene group; a substituted or unsubstituted heteroarylene group; a linear or branched, substituted or unsubstituted aralkylene group; a linear or branched, substituted or unsubstituted alkarylene group; or a silicium containing divalent group; and
X represents a z-valent group selected from the group consisting of a linear or branched, saturated or unsaturated, substituted or unsubstituted alkyl group; a linear or branched, saturated or unsaturated, substituted or unsubstituted heteroalkyl group; a saturated or unsaturated, substituted or unsubstituted cycloalkyl group; a saturated or unsaturated, substituted or unsubstituted heterocycloalkyl group; a substituted or unsubstituted aryl group; a substituted or unsubstituted heteroaryl group; a linear or branched, substituted or unsubstituted aralkyl group; a linear or branched, substituted or unsubstituted alkaryl group; or a silicium containing z-valent group.

4. The resin composition according to any one of the preceding claims,
wherein the at least one compound C2 has at least two thiol functional groups and at least one functional group selected from the group consisting of a silane, a siloxane, a carbonate, a carbamate, an ether and an ester,
and/or
wherein the ratio of the number of terminal alkyne functional groups to the number of thiol functional groups is from 1:1.8 to 1:2.25, in particular about 1:2, or wherein the ratio of the number of terminal alkyne functional groups to the number of thiol functional groups is smaller than 1:2, in particular from 1:2.01 to 1:2.3,
and/or
wherein the composition comprises from 10 to 90 wt.-% of the at least one compound C1,
and/or
wherein the composition comprises from 10 to 90 wt.-% of the at least one compound C2.

5. The resin composition according to any one of the preceding claims,
wherein the composition further comprises at least one initiator selected from the group consisting of a photoinitiator and a thermal initiator
and/or
wherein the composition further comprises at least one additive selected from the group consisting of a pigment, an inorganic filler, an organic filler and a stabilizer,
and/or
wherein the composition does not form a hydrogel,
and/or
wherein the composition is substantially solvent-free, in particular substantially water-free,
and/or
wherein the at least one compound C1 and the at least one compound C2 form one compound C3.

6. Use of a resin composition as defined in any one claims 1 to 5 as or in an ink.

7. A kit, in particular a kit-of-parts, comprising
at least one compound C1 having
(i) two terminal alkyne functional groups selected from the group consisting of propargyl, butynyl and pentynyl, and
(ii) one functional carbonate group; and
at least one compound C2 having at least two thiol functional groups.

8. Use of a kit as defined in claim 7 for preparing a resin composition for use as or in an ink.

9. A printing method comprising the steps of
providing a resin composition as defined in any one claims 1 to 5, the resin composition further comprising at least one initiator; and
directing an energy source to at least a part of the resin composition so as to cause polymerization of the at least a part of the resin composition and so as to obtain a polymer.

10. The printing method according to claim 9,
wherein the printing method is a two-dimensional printing method and the resin composition is provided on a substrate or wherein the printing method is a three-dimensional printing method, in particular one selected from the group consisting of stereolithography (SLA), two-photon absorption (TPA) polymerization, digital light processing (DLP), solid ground curing (SGC) and multi jet modeling (MJM),
and/or
wherein the at least one initiator comprises at least one photoinitiator; and the step of directing an energy source to at least a part of the resin composition comprises irradiating the at least a part of the resin composition with an energy-carrying activation beam, in particular electromagnetic radiation selected from the group consisting of ultraviolet radiation, visible light radiation, infrared radiation and microwave radiation,
and/or
wherein the at least one initiator comprises at least one thermal initiator; and the step of directing an energy source to at least a part of the resin composition comprises directing thermal energy to the at least a part of the resin composition,
and/or
wherein the printing method is a solvent-free printing method.

11. A polymer obtainable by the printing method according to claim 9 or 10.

12. An article comprising or formed from the polymer according to claim 11.

13. The article according to claim 12,
wherein the article is a medical device or a biomedical device, in particular selected from the group consisting of an implant, a bone substitute, a tissue substitute and a dental product,
and/or
wherein the article is substantially biodegradable or wherein the article is substantially non-biodegradable.

14. Use of a polymer according to claim 11 or of an article according to claim 12 or 13 in a medical or biomedical application.

15. The use according to claim 14, wherein the medical or biomedical application comprises any one selected from the group consisting of an implantation, a bone substitution or replacement, a tissue substitution or replacement, and a dental application.

## Patentansprüche

1. Eine Harzzusammensetzung aufweisend:
mindestens eine Verbindung C1, welche
(i) zwei endständige funktionelle Alkingruppen, welche aus der Gruppe ausgewählt sind, bestehend aus Propargyl, Butinyl und Pentinyl, und
(ii) eine funktionelle Carbonatgruppe hat; und
mindestens eine Verbindung C2, welche mindestens zwei funktionelle Thiolgruppen hat.

2. Die Harzzusammensetzung gemäß Anspruch 1, wobei mindestens eine der funktionellen Thiolgruppen eine Thiol Schutzgruppe aufweist, insbesondere wobei die Thiol Schutzgruppe ausgewählt ist aus der Gruppe, bestehend aus einer Acrylgruppe, einer Silylgruppe und einer Siloxylgruppe, und/oder wobei die Harzzusammensetzung ferner mindestens eine Fotosäure und/oder mindestens eine Fotobasis aufweist.

3. Die Harzzusammensetzung gemäß irgendeinem der vorangehenden Ansprüche,
wobei die mindestens eine Verbindung C2 mittels der folgenden allgemeinen Formel (VIII) dargestellt wird:
X-[L-S-Z]_{z} (XIII)
wobei
z eine ganze Zahl von 2 bis 1000 darstellt;
Z - unabhängig voneinander bei jedem Auftreten - Wasserstoff oder eine Thiol Schutzgruppe darstellt;
L - unabhängig voneinander bei jedem Auftreten - eine Einfachbindung oder eine zweiwertige Gruppe darstellt, welche ausgewählt ist aus der Gruppe, bestehend aus einer linearen oder verzweigten, gesättigten oder ungesättigten, substituierten oder unsubstituierten Alkylengruppe; einer linearen oder verzweigten, gesättigten oder ungesättigten, substituierten oder unsubstituierten Heteroalkylengruppe; einer gesättigten oder ungesättigten, substituierten oder unsubstituierten Cycloalkengruppe; einer gesättigten oder ungesättigten, substituierten oder unsubstituierten Heterocycloalkengruppe; einer substituierten oder unsubstituierten Arylengruppe; einer substituierten oder unsubstituierten Heteroarylengruppe; einer linearen oder verzweigten, substituierten oder unsubstituierten Aralkylengruppe; einer linearen oder verzweigten, substituierten oder unsubstituierten Alkarylengruppe; oder einem Silizium, welches eine zweiwertige Gruppe enthält; und
X eine z-wertige Gruppe darstellt, welche aus der Gruppe ausgewählt ist, bestehend aus einer linearen oder verzweigten, gesättigten oder ungesättigten, substituierten oder unsubstituierten Alkylgruppe; einer linearen oder verzweigten, gesättigten oder ungesättigten, substituierten oder unsubstituierten Heteroalkylgruppe; einer gesättigten oder ungesättigten, substituierten oder unsubstituierten Cycloalkylgruppe; einer gesättigten oder ungesättigten, substituierten oder unsubstituierten Heterocycloalkylgruppe; einer substituierten oder unsubstituierten Arylgruppe; einer substituierten oder unsubstituierten Heteroarylgruppe; einer linearen oder verzweigten, substituierten oder unsubstituierten Aralkylgruppe; einer linearen oder verzweigten, substituierten oder unsubstituierten Alkarylgruppe; oder einem Silizium, welches eine z-wertige Gruppe enthält.

4. Die Harzzusammensetzung gemäß irgendeinem der vorangehenden Ansprüche,
wobei die mindestens eine Verbindung C2 mindestens zwei funktionelle Thiolgruppen und mindestens eine funktionelle Gruppe hat, welche ausgewählt ist aus der Gruppe, bestehend aus einem Silan, einem Siloxan, einem Carbonat, einem Carbamat, einem Ether und einem Ester,
und/oder
wobei das Verhältnis der Anzahl von endständigen funktionellen Alkingruppen zu der Anzahl von funktionellen Thiolgruppen von 1:1.8 bis 1:2.25 ist, insbesondere ungefähr 1:2, oder wobei das Verhältnis der Anzahl von endständigen funktionellen Alkingruppen zu der Anzahl von funktionellen Thiolgruppen kleiner als 1:2 ist, insbesondere von 1:2.01 bis 1:2.3,
und/oder
wobei die Zusammensetzung von 10 bis 90 Gewichtsprozent der mindestens einen Verbindung C1 aufweist,
und/oder
wobei die Zusammensetzung von 10 bis 90 Gewichtsprozent der mindestens einen Verbindung C2 aufweist.

5. Die Harzzusammensetzung gemäß irgendeinem der vorangehenden Ansprüche,
wobei die Zusammensetzung ferner mindestens einen Initiator aufweist, welcher aus der Gruppe ausgewählt ist, bestehend aus einem Fotoinitiator und einem thermischen Initiator
und/oder
wobei die Zusammensetzung ferner einen Zusatzstoff aufweist, welcher ausgewählt ist aus der Gruppe, bestehend aus einem Pigment, einem anorganischen Füllstoff und einem Stabilisator,
und/oder
wobei die Zusammensetzung kein Hydrogel bildet,
und/oder
wobei die Zusammensetzung in Wesentlichen lösungsmittelfrei ist, insbesondere im Wesentlichen wasserfrei,
und/oder
wobei die mindestens eine Verbindung C1 und die mindestens eine Verbindung C2 eine Verbindung C3 bilden.

6. Verwendung einer Harzzusammensetzung wie in irgendeinem der Ansprüche 1 bis 5 definiert, als oder in einer Tinte.

7. Ein Kit, insbesondere ein Kit aus Teilen, aufweisend
mindestens eine Verbindung C1, welche
(i) zwei endständige funktionelle Alkingruppen, welche aus der Gruppe ausgewählt sind, bestehend aus Propargyl, Butinyl und Pentinyl, und
(ii) eine funktionelle Carbonatgruppe hat; und
mindestens eine Verbindung C2, welche mindestens zwei funktionelle Thiolgruppen hat.

8. Verwendung eines Kits wie in Anspruch 7 definiert zum Vorbereiten einer Harzzusammensetzung für eine Verwendung als oder in einer Tinte.

9. Ein Druckverfahren aufweisend die Schritte
Bereitstellen einer Harzzusammensetzung wie in irgendeinem der Ansprüche 1 bis 5 definiert, wobei die Harzzusammensetzung ferner mindestens einen Initiator aufweist; und
Richten einer Energiequelle auf zumindest einen Teil der Harzzusammensetzung, um eine Polymerisation von mindestens einem Teil der Harzzusammensetzung zu veranlassen, und um ein Polymer zu erhalten.

10. Das Druckverfahren gemäß Anspruch 9,
wobei das Druckverfahren ein zweidimensionales Druckverfahren ist und die Harzzusammensetzung auf einem Substrat bereitgestellt ist, oder wobei das Druckverfahren ein dreidimensionales Druckverfahren ist, insbesondere eines ausgewählt aus der Gruppe, bestehend aus Stereolithographie (SLA), Zwei Photonen Absorption (TPA) Polymerisation, digitaler Lichtverarbeitung (DLP), Solid Ground Curing (SGC) und Mehrstrahlmodellierung (MJM),
und/oder
wobei der mindestens eine Initiator mindestens einen Fotoinitiator aufweist; und der Schritt des Richtens einer Energiequelle auf zumindest einen Teil der Harzzusammensetzung ein Bestrahlen des zumindest einen Teils der Harzzusammensetzung mit einem energietragenden Aktivierungsstrahl aufweist, insbesondere elektromagnetische Strahlung ausgewählt aus der Gruppe, bestehend aus ultravioletter Strahlung, sichtbarer Lichtstrahlung, Infrarotstrahlung und Mikrowellenstrahlung,
und/oder
wobei der mindestens eine Initiator mindestens einen thermischen Initiator aufweist; und der Schritt des Richtens einer Energiequelle auf zumindest einen Teil der Harzzusammensetzung ein Richten von thermischer Energie auf den zumindest einen Teil der Harzzusammensetzung aufweist,
und/oder
wobei das Druckverfahren ein lösungsmittelfreies Druckverfahren ist.

11. Ein Polymer erhältlich mittels des Druckverfahrens gemäß Anspruch 9 oder 10.

12. Ein Artikel aufweisend oder gebildet aus dem Polymer gemäß Anspruch 11.

13. Der Artikel gemäß Anspruch 12,
wobei der Artikel ein medizinisches Gerät oder ein biomedizinisches Gerät ist, insbesondere ausgewählt aus der Gruppe, bestehend aus einem Implantat, einem Knochenersatz, einem Gewebeersatz und einem zahnmedizinischen Produkt,
und/oder
wobei der Artikel im Wesentlichen biologisch abbaubar ist, oder wobei der Artikel im Wesentlichen nicht biologisch abbaubar ist.

14. Verwendung eines Polymers gemäß Anspruch 11 oder eines Artikels gemäß Anspruch 12 oder 13 in einer medizinischen oder biomedizinischen Anwendung.

15. Die Verwendung gemäß Anspruch 14, wobei die medizinische oder biomedizinische Anwendung irgendeines ausgewählt aus der Gruppe aufweist, bestehend aus einer Implantierung, einem Knochenersatz oder Ersatzmaterial, einem Gewebeersatz oder Ersatzmaterial, und einer zahnmedizinischen Anwendung.

## Revendications

1. Composition de résine comprenant :
au moins un composé C1 ayant
(i) deux groupes fonctionnels alcyne terminaux sélectionnés dans le groupe consistant en le propargyle, le butynyle et le pentynyle, et
(ii) un groupe carbonate fonctionnel ; et
au moins un composé C2 ayant au moins deux groupes fonctionnels thiol.

2. Composition de résine selon la revendication 1, dans laquelle au moins un des groupes fonctionnels thiol comprend un groupe protecteur de thiol, en particulier dans laquelle le groupe protecteur de thiol est sélectionné dans le groupe consistant en un groupe acyle, un groupe silyle et un groupe siloxyle, et/ou dans laquelle la composition de résine comprend en outre au moins un photoacide et/ou au moins une photobase.

3. Composition de résine selon l'une quelconque des revendications précédentes, dans laquelle au moins un composé C2 est représenté par la formule générale (XIII) suivante :
X-[L-S-Z]_{z} (XIII)
dans laquelle
z représente un nombre entier de 2 à 1000 ;
Z représente - indépendamment les uns des autres à chaque occurrence - un hydrogène ou un groupe protecteur de thiol ;
L représente - indépendamment les uns des autres à chaque occurrence - une liaison simple ou un groupe divalent sélectionné dans le groupe consistant en un groupe alkylène linéaire ou ramifié, saturé ou insaturé, substitué ou non substitué ; un groupe hétéroalkylène linéaire ou ramifié, saturé ou insaturé, substitué ou non substitué ; un groupe cycloalkylène saturé ou insaturé, substitué ou non substitué ; un groupe hétérocycloalkylène saturé ou insaturé, substitué ou non substitué ; un groupe arylène substitué ou non substitué ; un groupe hétéroarylène substitué ou non substitué ; un groupe aralkylène linéaire ou ramifié, substitué ou non substitué ; un groupe alkarylène linéaire ou ramifié, substitué ou non substitué ; ou un groupe divalent contenant du silicium ; et
X représente un groupe z-valent sélectionné dans le groupe consistant en un groupe alkyle linéaire ou ramifié, saturé ou insaturé, substitué ou non substitué ; un groupe hétéroalkyle linéaire ou ramifié, saturé ou insaturé, substitué ou non substitué ; un groupe cycloalkyle saturé ou insaturé, substitué ou non substitué ; un groupe hétérocycloalkyle saturé ou insaturé, substitué ou non substitué ; un groupe aryle substitué ou non substitué ; un groupe hétéroaryle substitué ou non substitué ; un groupe aralkyle linéaire ou ramifié, substitué ou non substitué ; un groupe alkaryle linéaire ou ramifié, substitué ou non substitué ; ou un groupe z-valent contenant du silicium.

4. Composition de résine selon l'une quelconque des revendications précédentes, dans laquelle au moins un composé C2 présente au moins deux groupes fonctionnels thiol et au moins un groupe fonctionnel sélectionné dans le groupe consistant en un silane, un siloxane, un carbonate, un carbamate, un éther et un ester,
et/ou
dans laquelle le rapport entre le nombre de groupes fonctionnels alcyne terminaux et le nombre de groupes fonctionnels thiol est de 1:1,8 à 1:2,25, en particulier d'environ 1:2, ou dans laquelle le rapport entre le nombre de groupes fonctionnels alcyne terminaux et le nombre de groupes fonctionnels thiol est inférieur à 1:2, en particulier de 1:2,01 à 1:2,3,
et/ou
dans laquelle la composition comprend de 10 à 90 % en poids d'au moins un composé C1,
et/ou
dans laquelle la composition comprend de 10 à 90 % en poids d'au moins un composé C2.

5. Composition de résine selon l'une quelconque des revendications précédentes,
dans laquelle la composition comprend en outre au moins un initiateur sélectionné dans le groupe consistant en un photoinitiateur et un initiateur thermique
et/ou
dans laquelle la composition comprend en outre au moins un additif sélectionné dans le groupe consistant en un pigment, une charge inorganique, une charge organique et un stabilisant,
et/ou
dans laquelle la composition ne forme pas d'hydrogel,
et/ou
dans laquelle la composition est sensiblement exempte de solvant, en particulier sensiblement exempte d'eau,
et/ou
dans laquelle au moins un composé C1 et au moins un composé C2 forment un composé C3.

6. Utilisation d'une composition de résine telle que définie dans l'une quelconque des revendications 1 à 5 comme ou dans une encre.

7. Kit, en particulier kit de parties, comprenant au moins un composé C1 ayant
(i) deux groupes fonctionnels alcyne terminaux sélectionnés dans le groupe consistant en le propargyle, le butynyle et le pentynyle, et
(ii) un groupe carbonate fonctionnel ; et
au moins un composé C2 ayant au moins deux groupes fonctionnels thiol.

8. Utilisation d'un kit tel que défini dans la revendication 7 pour préparer une composition de résine à utiliser comme ou dans une encre.

9. Procédé d'impression comprenant les étapes suivantes
la fourniture d'une composition de résine telle que définie dans l'une quelconque des revendications 1 à 5, la composition de résine comprenant en outre au moins un initiateur ; et
l'orientation d'une source d'énergie vers au moins une partie de la composition de résine de manière à provoquer la polymérisation d'au moins une partie de la composition de résine et de manière à obtenir un polymère.

10. Procédé d'impression selon la revendication 9, dans lequel le procédé d'impression est un procédé d'impression bidimensionnel et la composition de résine est fournie sur un substrat ou dans lequel le procédé d'impression est un procédé d'impression tridimensionnel, en particulier un procédé sélectionné dans le groupe consistant en une stéréolithographie (SLA), une polymérisation par absorption à deux photons (TPA), un traitement numérique de la lumière (DLP), un durcissement au sol solide (SGC) et un modelage à jets multiples (MJM),
et/ou
dans lequel au moins un initiateur comprend au moins un photoinitiateur ; et l'étape d'orientation d'une source d'énergie vers au moins une partie de la composition de résine comprend l'irradiation d'au moins une partie de la composition de résine avec un faisceau d'activation porteur d'énergie, en particulier un rayonnement électromagnétique sélectionné dans le groupe consistant en un rayonnement ultraviolet, un rayonnement de lumière visible, un rayonnement infrarouge et un rayonnement de micro-ondes,
et/ou
dans lequel au moins un initiateur comprend au moins un initiateur thermique ; et l'étape d'orientation d'une source d'énergie vers au moins une partie de la composition de résine comprend l'orientation d'une énergie thermique vers au moins une partie de la composition de résine,
et/ou
dans lequel le procédé d'impression est un procédé d'impression exempt de solvant.

11. Polymère pouvant être obtenu par le procédé d'impression selon la revendication 9 ou 10.

12. Article comprenant ou formé à partir du polymère selon la revendication 11.

13. Article selon la revendication 12,
dans lequel l'article est un dispositif médical ou un dispositif biomédical, en particulier sélectionné dans le groupe consistant en un implant, un substitut osseux, un substitut tissulaire et un produit dentaire,
et/ou
dans lequel l'article est sensiblement biodégradable ou dans lequel l'article est sensiblement non biodégradable.

14. Utilisation d'un polymère selon la revendication 11 ou d'un article selon la revendication 12 ou 13 dans une application médicale ou biomédicale.

15. Utilisation selon la revendication 14, dans laquelle l'application médicale ou biomédicale comprend l'une quelconque sélectionnée dans le groupe consistant en une implantation, une substitution ou un remplacement osseux, une substitution ou un remplacement tissulaire, et une application dentaire.
